# EUROPEAN PATENT APPLICATION

(11) **EP 4 591 806 A1**
(43) Date of publication of application: **30.07.2025**
(21) Application number: 24154099.6
(22) Date of filing: 26.01.2024
(51) Int. Cl.: A61B 17/16

(54) **ORTHOPEADIC REAMER**

(71) Applicant: Beck, Steffen, 80469 München (DE)
(72) Inventor: Beck, Steffen, 80469 München (DE)
(74) Representative: Hoefer & Partner Patentanwälte mbB

(57) **Abstract**

The invention concerns an orthopeadic reamer (1), preferably acetabular reamer, for milling a hemispherical cavity (901) in a bone (900), preferably adapted for single use, comprising a body (2), having a hemispherical portion (3) spanning a cavity (4), preferably to store cut bone debris, adapted for attachment of a drilling machine (700), preferably via a connector portion (5) of the body, to rotate the reamer (1) around a rotation axis (6), and at least one ring-shaped blade element (7) having a curved cutting section (8) with a sharp cutting edge (9), wherein the ring-shaped blade element (7), preferably each ring-shaped blade element, is embedded in the hemispherical portion (3), and wherein a blade axis (10), preferably for each ring-shaped blade element, is defined perpendicular to a radius (35) of the ring-shaped blade element (7), and the blade axis (10), preferably of each ring-shaped blade element, is inclined to the rotation axis (6).

## Description

The invention concerns orthopeadic reamers, preferably acetabular reamers, for milling a hemispherical cavity in a bone, preferably adapted for single use; methods for manufacturing an orthopeadic reamer; an orthopeadic drilling machine; and an arrangement for milling a hemispherical cavity in a bone.

In the prior art, there are various patents related to acetabular reamers used in orthopaedic surgeries. These reamers typically consist of a hemispherical metal shell with perforated holes acting as cutting edges, e.g. US 4,023,572 A. The design is often referred to as 'cheese grater' design. One reamer might have approximately 30 to 40 of said cutting edges. Forming and sharpening these cutting edges is often a manual task leading to high manufacturing costs.

A second commonly used design are reamers with cutting sockets (US 4,131,116 A) The reamers are made of metal and of general hemispherical design. Multiple of these sockets are placed often symmetrical along the surface of the spherical reamer. The non-leading edge of each socket is elevated above the sphere of the reamer and sharpened in order to remove bone. These reamers are commonly formed from sheet metal bend into hemispherical shape by deep drawing or rotation drawing. The sockets are CNC machined.

Hence both cheese grater design reamers and reamers with cutting sockets are used in clinical practice for multiple procedures. Reusable reamers require sufficient cleaning and sterilization after each use adding costs to the hospital and further increase the cycle time of sterilizing the instrumentation set required for orthopaedic surgery. As autoclave space is limited in most hospitals there is a desire for the use of more single use products. Also reusable devices might increase the risk of cross contamination. Last reusable orthopaedic reamers wear with each use. A dull reamer might increase the time required for reaming the bone, decrease the quality of the reamed surface and increase the heat generated during reaming which has a negative effect on tissue. Hence it is desired to have a sharp and sterile single use reamer which is economically viable due to low manufacturing costs. Conventionally reaming of the acetabulum is started with a reamer smaller the size of the hip joint to define the depth of the ream before constantly increasing the size of the reamer until 1-2mm below the size of the final hip implant.

This technique provides a high degree of control of the reaming process however it comes with the disadvantage that the hip joint centre is often medialized. A second technique known as peripheral reaming is therefore desired where the acetabulum is prepared with a reamer larger than the native hip joint. Due to the larger size of the reamer the reamer is primarily contacting the hip joint at an area close to its equator. Conventional reamer systems do not cut well at the equator area making peripheral reaming less controllable. Hence a reamer with improved peripheral cutting characteristics is desired.

Robotic systems as the one presented in EP 2 482 748 A2, US 8 498 744 B2, are used during hip replacement surgery to control e.g. the reaming process. The robot therefore has an electromechanical reamer drill attached to a robotic arm. The position and orientation of the reamer drill, robotic arm and patient bone in 3D space are further tracked by a stereoscopic navigation system connected to a computation unit. The computation unit has the desired position and orientation of the hip implant in respect to the hip bone stored as input value. Based on the current position of the reamer in respect to the desired state the computational unit controls the power of the reamer drill. Further it controls the robotic arm in such a way that the reamer drill can only be moved by the operator within a desired three-dimensional space. The power trigger and orientation of the reamer drill within the allowed space is controlled by the operator. Such systems are referred to as passive controlled robotic arm systems. The use of passive controlled robotic arm systems is constantly growing. However a drawback of these systems is the use of a large robotic arm which limits the available space the surgeon team can operate. Also a large system requires significant set up time before surgery and often requires an additional operator. Robotic arm systems are also expensive and the cost-benefit assessment of these systems is subject of discussion in the literature.

It is hence an object of the invention to have a reamer, which allows for low-cost manufacturing, superior cutting performance, high mechanical stiffness and accurate manufacturing and assembly to achieve an accurate spherical ream.

In the following disclosure of the invention, all features disclosed under a point (e.g. "(1)") can preferably be combined with any feature disclosed under the same point and/or under the any referenced point(s), unless otherwise mentioned.

### (1) Reamer with embedded ring-shaped blade element(s):

The invention comprises an orthopeadic reamer, preferably acetabular reamer, for milling a hemispherical cavity in a bone, preferably adapted for single use, comprising a body, (i) having a hemispherical portion spanning a cavity, preferably to store cut bone debris; the hemispherical portion is adapted for attachment of a drilling machine (700) to rotate the reamer (1) around a rotation axis (6); preferably the body comprises a connector portion adapted for the attachment of the drilling machine to rotate the reamer around a rotation axis. Further, the reamer comprising at least one ring-shaped blade element (i) having a curved cutting section with a sharp cutting edge, (ii) wherein the ring-shaped blade element, preferably each ring-shaped blade element, is embedded in the hemispherical portion, and (iii) wherein a blade axis, of the ring-shaped blade element is defined perpendicular to the radius of the ring-shaped blade element, and the blade axis, preferably of each ring-shaped blade element, is inclined to the rotation axis.

The embodiment under point (1) discloses "ring-shaped blade element(s)", embedded as separate part(s) in the hemispherical portion of the body. A further embodiment disclosed below under point (2) discloses "curved blade element(s)" as integral part(s) of the hemispherical portion (the curved blade element(s) and the hemispherical portion is one part). Any optional feature and advantage referring to "blade element(s)" and/or to the "blade axis" disclosed herein preferably apply to both the "ring-shaped blade element(s)" and the "curved blade element(s)". All optional features disclosed herein referring to the "connector portion" apply to the embodiment under point (1) and to the embodiment under point (2), unless otherwise stated in detail.

As will be described, the reamer preferably comprises only one blade element. However, there are also embodiments with several blade elements, preferably two, three, or four embedded ring-shaped blade elements or integral curved blade elements, so that the features given below with regard to the blade element - unless otherwise mentioned - preferably apply to all blade elements.

The inventive reamer is preferably designed for a low-cost disposable/single-use. The reamer is used for cutting bone during orthopaedic procedures like hip replacement procedures. The reamer allows for milling of a spherical cavity with high dimensional accuracy. A typical application of such a reamer is the preparation of a ball and socket joint such as the hip by reaming the acetabular cavity to a hemispherical shape (also called cutting hemisphere) with a desired diameter for implanting a hemispherical shaped implant. For the sake of simplicity, the following refers to the reamer cutting bone, although this always means that cartilage or tissue can also be cut.

Preferably, the connector portion and its connection to the hemispheric portion is rigid, so that the connector portion and the hemispheric portion rotate around the common rotation axis without wobbling relative to each other.

Preferably, the hemispherical portion extends from its apex to its equator. The connector portion is preferably located in the area of the equator. Preferably, the cavity of the reamer is formed between the inside of the hemispherical portion and the connector portion, which is in the area of the equator. The hemispherical portion preferably forms a continuous hemispherical surface over the entire circumference and from the apex to the equator in order to guide and support the reamer relative to the bone. However, a minimally invasive design is also possible in which the hemispherical shape is not continuous so that the reamer is narrower and can therefore be inserted into the patient's body through smaller openings.

The body and the connector portion can be attached to each other or manufactured as one part. Thereby, the connector portion can be positioned inside the body, so that the connector portion does not influence the hemispherical outer shape of the reamer. However, if the connector portion protrudes from the equator of hemispherical portion, it is preferred that the outer shape of the connector portion has smaller diameter than the hemispherical portion. This is different to conventional reamers which have an outer surface of cylindrical shape below the equator with same diameter as the hemispherical portion. The preferred smaller diameter of the inventive reamer has the advantage that the reamer can be rotated freely within the prepared joint socket without the cylindrical shape to leaver the reamer out of the joint socket.

Preferably, the rotation axis of the reamer intersects the apex of the hemispherical portion and the center point of the connector portion.

As mentioned, the blade axis is inclined to the rotation axis. Thereby, the blade element is preferably adapted in size and position, such that the cutting edge of the cutting section moves on a virtual cutting sphere when the reamer is rotated around its rotation axis. The design follows the geometrical principle that the intersection of a sphere and a cylinder is a circle when the rotation axis of the cylinder (the blade axis) intersects the midpoint of the sphere (which is the midpoint of the hemispherical portion).

Preferably, the blade axis intersects the midpoint of the hemispherical portion.

To cut a half sphere in the bone, in particular with only one blade element, the cutting edge of the blade element should intersect the apex and the equator of the cutting sphere. When using more than one blade elements, the cutting edge of one blade element can intersect the apex and the cutting edge of another blade element can intersect the equator. The term "intersects the apex" also includes that the respective cutting edge can have a small distance from the apex, preferably of 3 mm or less, more preferably of 2 mm or less. The cutting edge can have this small distance from the apex of the hemispherical portion; the apex of the cutting sphere (lowest point of the half sphere in bone) is nevertheless cut due to a wobbling movement of the overall system consisting of the drilling machine and the reamer.

Preferably, the ring-shaped blade element is a closed ring extending over a ring angle of 360° around the blade axis, or is an open ring extending over a ring angle of at least 200°, preferably at least 250°, around the blade axis.

The ring-shaped blade element can also be termed cylindrical-shaped blade element. The ring angle is defined around the blade axis.

The closed ring can be manufactured by joining the ends of an element, for example by soldering or welding. However, it is also preferable that the closed ring in its closed form is cut from a tube or turned or milled from a body.

The forces applied by the surgeon and reamer drill influence the cutting performance of the reamer. The forces applied by the operator are mainly applied in axial direction pushing the reamer onto the bone surface while the reamer drill applies a torsion force. It is beneficial to keep both axial and torsional force reasonably low by the inventive described reamer design parameters to prevent force introduced damage to the bone. However a reasonable amount of axial and torsional forces are required to perform the reaming action. It is important for the reamer to mechanically resist these forces without brakeage and significant deformation which would alter the dimensions of the reamed cutting sphere. The closed ring design has significantly higher bending and torsion resistance compared to an open ring design or a flat blade design given the same blade thickness. Hence a smaller blade thickness can be chosen compared to the other designs. This is beneficial as the reamer is intended to be a single use instrument. Less material does result in lower part costs, lesser disposal costs and a better economical footprint of the device.

Preferably, the ring-shaped blade element is circular or elliptical.

Preferably, the cutting section of the ring-shaped blade element merges into a support section of the ring-shaped blade element, so that the circumference of the ring-shaped blade element is formed by the cutting section and by the support section.

Preferably, the support section is so deeply embedded in the body that the body sections close to the support section contact the bone, wherein the support section preferably does not contact the bone.

Preferably, the area of the cross section of the ring-shaped blade element is at most 100 qmm (square millimeter), preferably at most 50 qmm; wherein the area is measured at the thickest point of the cutting section, preferably at the thickest point of the ring-shaped blade element. The area of the cross section is considered in a plane in which the blade axis lies. If the plane intersects the ring-shaped blade element twice, only one of the two intersections count as "area of the cross section".

Preferably, the cutting section of the blade element extends over a section angle between 100° and 220°, preferably between 160° and 200°, around the blade axis.

Although different sections are defined on the blade element, the blade element is preferably a one-piece component.

The support section has preferably no cutting edge and is preferably positioned as to have no contact to the bone. The support section supports the stiffness of the blade element and the attachment of the ring-shaped blade element to the body.

When using the reamer, it is rotated so that the support section is at the front and the cutting section is at the rear in the direction of rotation. This is why the support section can also be referred to as leading section and the cutting section as non-leading section. For the same reason, the cutting edge has a leading edge or side and a non-leading (also called trailing) edge or side. As the blade element is ring-shaped, the cutting section and the support section are curved. Consequently, the cutting section has a concave side that points forwards in the direction of rotation when the reamer rotates. The cutting edge is aligned in such a way that it cuts.

Preferably, the hemispherical portion, preferably the entire body, is made of plastic and the blade element is made of metal or ceramic.

Preferably, the ring-shaped blade element is glued and/or snap fitted and/or press fitted and/or welded to the hemispherical portion. If the ring-shaped blade element is welded on, it is preferably fixed by ultrasonic welding or hot air welding, whereby the plastic of the hemispherical portion is melted near the ring-shaped blade element in order to fix the blade element.

Alternative preferable, the ring-shaped blade element is overmolded by the injection molded hemispherical portion or is overprinted by the 3D-printed hemispherical portion. Overmolded means that the ring-shaped blade element was inserted into the injection mold and is partially covered by the plastic material of the hemispherical portion. Overprinted means that the ring-shaped blade element was inserted into the 3D printing machine and is partially covered by the plastic material of the hemispherical portion.

Instead of the using plastic, preferably, the hemispherical portion, preferably the entire body, is made of metal and the ring-shaped blade element is made of metal or ceramic. Preferably, the ring-shaped blade element is soldered and/or welded and/or snap fitted and/or press fitted to the hemispherical portion.

Preferably, the reamer is designed as one blade reamer having only the one ring-shaped blade element. Preferably, the reamer has no other cutting elements and/or no other grinding elements.

Alternative preferably, the reamer is designed as two blade reamer having only two of the ring-shaped blade elements. Preferably, the reamer has no other cutting elements and/or no other grinding elements. Alternative preferably, the reamer is designed as three blade reamer having only three of the ring-shaped blade elements. Preferably, the reamer has no other cutting elements and/or no other grinding elements. Alternative preferably, the reamer is designed as four blade reamer having only four of the ring-shaped blade elements. Preferably, the reamer has no other cutting elements and/or no other grinding elements.

Preferably each ring-shaped blade element is designed as a closed ring. Preferably the blade axis of all ring-shaped blade elements are inclined to each other and are inclined to the rotation axis. Preferably, the two or three or four ring-shaped blade elements do not intersect witch each other. When using the reamer, however, the two or three or four ring-shaped blade elements can partially cut the same area of the bone. Preferably, the curved recess (mentioned below) is provided at each of the two or three or four ring-shaped blade elements. Preferably, the two or three or four ring-shaped blade elements are arranged next to each other and not inside each other.

Preferably the cutting sections of the ring-shaped blade elements of a two, three or four blade reamer are not evenly (symmetrically) distributed around the reamer rotation axis but placed in an angle section spanning around the rotation axis having less than 180° (the cutting sections of all ring-shaped blade elements are arranged on one half of the hemispherical portion), preferably less than 160°, and more preferably less than 140°. Arranged as such the surface of the hemispherical portion outside the angle section (opposing area) is in contact with the bone during reaming and especially opposed to each cutting section (180° rotation around rotation axis) there is an area of the hemispherical portion which is in contact with the bone. This has the advantage of a smooth ream and higher manufacturing accuracy, similar to the single cutting area, which will be explained below.

According to an alternative definition, the center points of the ring-shaped blade elements are placed in the angle section spanning around the rotation axis having less than 180°, preferably less than 160°, and more preferably less than 140°

Preferably, the reamer comprising an X-ray absorber in the body. Preferably the X-ray absorber is a part, in particular an elliptic ring, or the X-ray absorber is made by a X-ray absorbing material added to the plastic compound of the body. It is common practice during surgery to check correct position and orientation of the reamer on a fluoroscopic image. As the reamer body and/or connector portion can be manufactured from plastic its visibility within the fluoroscopic image is limited. To allow better judgement a ring as X-ray reflector of a material well visible in X-ray images such as metal might be added, e.g. at the reamer equator. Depending on the orientation of the reamer to the fluoroscopic device the ring is shaped as an ellipse whose shape allows the surgeon to judge the orientation of the reamer in respect to the patient's anatomy. Alternatively, a contrast medium as X-ray reflector might be added to the plastic compound to increase visibility of the whole reamer.

### (2) Reamer with integral curved blade element(s):

The invention comprise an orthopeadic reamer, preferably acetabular reamer, for milling the hemispherical cavity in the bone, preferably adapted for single use, comprising the body, (i) having the hemispherical portion spanning the cavity, preferably to store cut bone debris, (ii) and having the connector portion adapted for attachment of the drilling machine to rotate the reamer around a rotation axis, and comprising at least one curved blade element (i) having the curved cutting section with the sharp cutting edge, (ii) wherein the curved blade element, preferably each curved blade element, is integral part of the hemispherical portion, and (iii) wherein the blade axis of the curved blade element is defined perpendicular to the radius of the curved blade element, and the blade axis, preferably of each curved blade element, is inclined to the rotation axis. The hemispherical portion and the at least one curved blade element is one part, preferably formed out of one metal part. Preferably, the at least one curved blade element is formed by a recess punched in the hemispherical body. Thereby the cutting edge is preferably elevated above the surface of the hemisphere by the desired cutting depth.

Preferably, the reamer is designed as one blade reamer having only the one curved blade element. Preferably, the reamer has no other cutting elements and/or no other grinding elements.

Alternative preferably, the reamer is designed as two blade reamer having only two of the curved blade elements. Preferably, the reamer has no other cutting elements and/or no other grinding elements. Alternative preferably, the reamer is designed as three blade reamer having only three of the curved blade elements. Preferably, the reamer has no other cutting elements and/or no other grinding elements. Alternative preferably, the reamer is designed as four blade reamer having only four of the curved blade elements. Preferably, the reamer has no other cutting elements and/or no other grinding elements.

Preferably the blade axis of all curved blade element are inclined to each other and are inclined to the rotation axis. Preferably, the two or three or four curved blade elements do not intersect witch each other. When using the reamer, however, the two or three or four curved blade elements can partially cut the same area of the bone. Preferably, the curved recess (mentioned below) is provided at each of the two or three or four curved blade elements. Preferably, the two or three or four curved blade elements are arranged next to each other and not inside each other.

### (3) Further optional feature(s) of the orthopeadic reamer according to point (1) or (2), or any optional feature disclosed under point (1) or (2):

Preferably, a single cutting area is defined on the hemispherical portion of the body, wherein only one blade element is disposed in the single cutting area - even if the reamer comprises two or three or four of the blade elements. Preferably no other cutting elements and/or no grinding elements are disposed in the single cutting area.

The single cutting area is a full circumference ring area that extends from the equator of the hemispherical portion in direction of the apex. A height of the single cutting area is measured from the equator in direction of the apex parallel to the rotation axis of the reamer. The height is at least 0,2*R, preferably at least 0,25*R, with R being the radius of the hemispherical portion. For the one blade element design, the height can be R or almost R.

Preferably, the single cutting area is designed as a smooth, spherical surface to support and guide the reamer gliding on the bone.

An advantage of the single cutting area is, that not only the blade element is in contact with the bone but also part of the single cutting area. Hence the dimension of the cut sphere is defined by the relative distance of the cutting edge of the blade element(s) and part of the single cutting area. Prior art reamer designs have multiple cutting elements equally spaced about the reamer surface being in contact with the bone and hence their relative position to each other defines the dimensions of the cut sphere. Using the inventive reamer body's outer surface as size defining parameter for the reamed sphere diameter significantly increases the dimensional accuracy of the ream as the tolerance chain only contains manufacturing and assembly tolerances of the blade element(s) and the reamer body - compared to a multi-blade reamer design where the tolerance chain contains the manufacturing and assembly tolerances of all cutting elements and the reamer body connecting the cutting elements. Preferably, there is a difference in radius of the blade's cutting sphere and the hemispherical portion so that the cutting edge protrudes the hemispherical portion by a distance d_{cut} which is the maximum cutting depth of the reamer blade. To achieve this protrusion, the hemispherical portion might be sunk in by the distance d_{cut} in an area around the cutting edge. Alternatively and preferably the radius of the cutting sphere might have a radius of length r+d_{cut}/2 (r being the radius of the desired hemisphere in the bone) and the hemisphere portion a radius of length r-d_{cut}/2 (r being the radius of the desired hemisphere in the bone). As the reamer self centers within the bone the reamer rotates within the acetabulum with a small wobbling motion and thereby cuts a hemisphere of radius r. This option has the advantage that the hemispherical portion is of a constant radius and does not need to be sunk in which adds complexity to the manufacturing process.

The design with single cutting area was found to increases the 'smoothness' of the ream experienced by the operator especially during peripheral reaming, limit's potential stalling of the reamer, and lowers cutting vibrations. This might be due to the following behavior of multi blade reamers having more than four blades and/or having no single cutting area and/or having opposing blades and/or having symmetrically/evenly distributed blades. Such multi blade reamers rotates around its primary rotation axis. Each blade moves in a circle around this primary rotation axis, hence having a first tangential movement vector. Likewise to a wheel rolling on ground a multi blade reamer might additionally rotate around a second rotation point when one of the blades is experiencing a high resistance during cutting. This second rotation point is the contact point of the blade and the bone. With the reamer rotating also around this second rotation point a second movement vector is introduced at the other blades. Hence these blades no longer solely move tangential along the bone surface but are forced to either lift off or cut deeper into the bone surface. This results in higher cutting resistance and potential stalling of the reamer. Inventive reamers can be designed to have no opposing reamer blades in the single cutting area which prevents cutting too deep into the bone, vibrations, and stalling of the reamer.

Further, the inventive reamer might be easier to insert and remove from the joint socket without damaging surrounding soft tissue as the reamer can be oriented by the operator as such that the non-cutting part of the reamer is facing critical soft tissue structures. Also handling of the reamer is simplified as a larger surface is available to safely grab the reamer without the risk of touching the sharp cutting edge of the reamer.

Preferably, the hemispherical portion having at least one curved recess, preferably a curved slit formed recess. The curved recess can also be termed curved notch.

Preferably, the curved recess is adjacent and in cutting direction directly in front of the cutting edge, to transport the cut bone debris into the cavity. Preferably wherein the hemispherical surface of the hemispherical portion is, except for the recess, closed.

Preferably, a section, referred to as flap, of the hemispherical portion on the concave side of the curved recess is adjustable in order to adjust the cutting depth of the blade element. The flap is preferably the portion inside the ring-shaped blade element or on the concave side of the curved blade element.

Preferably, when using the reamer, the flap is bendable inward by axial force on the reamer to increase the cutting depth in dependency of the axial force. Preferably, whereby the flap - if no axial force is applied - protrudes over the cutting edge.

Preferably, the flap has a desired stiffness against the external radial forces which occur when the operator is pressing the reamer in axial direction (axial force) against the bone surface. The flap is preferably designed as such that it's outer surface is elevated as such that it has the same or a larger radial distance to the reamer centre as the cutting edge of the blade element. When external forces are present the flap acts as a leaf spring where the larger the radial forces the smaller the distance to the reamer centre and hence the greater the cutting depth which is defined by the radial distance of the cutting edge and the outer surface of the flap. Designed as such the reamer cuts little bone when the operator applies limited axial forces and cuts more aggressively when the operator applies larger axial forces.

Preferably, the reamer comprising an adjustment mechanism, preferably positioned in the cavity of the hemispherical portion, wherein the adjustment mechanism is adapted to lift and/or lower the flap relative to the blade element in order to adjust the cutting depth of the blade element.

Instead of the movable/bendable flap, preferably, the reamer has a cut preventing component, and an adjustment mechanism, wherein the cut preventing component is movable through the recess or a separate opening in the hemispherical portion by the adjustment mechanism, so that the cut preventing component can overtop the cutting edge to prevent the reamer from cutting; thereby preferably adjusting the cutting depth to zero. Thereby, it also possible that the cut preventing component is not overtopping the cutting edge but gluts the blade when it is moved in close proximity to the cutting edge, so that it blocks/hinders bone chips from entering into the recess and hence temporarily glut the cutting section hindering the blade element from cutting as long as the cut prevention component is in the lift up state.

In another preferred option, the adjustment mechanism is adapted to alter the height of the ring blade element so that the cutting edge is or is not protruding the hemispherical portion of the reamer.

For the movable flap and for the cut preventing component, preferably, the adjustment mechanism has a control arrangement, which is adapted to be operated by a control movement of the control element of the drill machine.

Preferably, the sharp cutting edge of all mentioned blade elements having following angles, wherein the spherical surface on which the tip of the cutting edge moves when the reamer is used is referred to as sliding plane, and the cutting edge is positioned between the sliding plane and the normal vector of the sliding plane:
(i) clearance angle between the sliding plane and the outward facing side of the cutting edge;
(ii) a rake angle between the normal vector of the sliding plane and the inward facing side (blades leading face) of the cutting edge; the rake angel, in case of the curved cutting section, preferably increases, in particular by at least 20°, in the direction of the apex; the rake angle is preferably at all points less than 90°, in particular less than 70°, and/or is preferably at all points larger than 0°, in particular between 10° and 30°. A smaller rake angle is used to cut harder material. A rake angle close to 0° will result in scraping rather than cutting off the material which increases heat generation. For the ring-shaped or curved blade element the rake angle preferably varies along its latitude increasing from the equator towards the apex. This is beneficial as hard bone due to osteophytes might be present closer to the reamer apex.
(iii) and a blade angle between the inward facing side and the outward facing side of the cutting edge; the blade angle is preferably between 30° and 80°, more preferably between 50° and 70°. The blade angle is preferably designed as such that a large enough clearance angle is created which is required to allow penetration of the blade into the material.

The sharpness of the cutting edge is important to reduce the resistance the blade element experiences when penetrating the material. A sharper edge will minimize the cutting momentum resulting in less force to be counteracted by the operator and reducing the risk of stalling of the reamer drill. The cutting edge can well be sharpened e.g. on a lathe using a grinding tool or their like. As the reamer is intended to be used as a single use instrument a sharp cutting edge is given all the time.

The cutting power is a function of the reamer blade's rotation/movement speed relative to the bone material. The movement speed increases from the hemispheric portion apex towards the equator as the distance towards the rotation axis is increasing. Hence the cutting power does increase towards the equator. However as more bone material is removed closer to the apex a more unified cutting force is desired. The curved cutting section achieves a more unified cutting power as the angle of attack, the angle between the cutting edge and the movement vector, preferably decreasing towards the equator. A smaller angle of attack leads to lower cutting power.

Sufficient control of the cutting depth is beneficial as it has a major effect on the force/momentum required to remove a bone chip. It is beneficial to minimize the momentum required to perform reaming hence a small cutting depth is desirable. The distance between the cutting edge of the blade element and the outer surface of the hemispheric portion measured in radial direction defines the maximum cutting depth of the reamer. A cutting depth of 0,05mm to 0,5mm, preferably 0,1mm to 0,3mm, was found to generate a smooth cut without vibrations and forming of thin bone chips which seamlessly move through the recess into the cavity of the reamer body where they are stored.

Therefore, the cutting depth of the reamer is preferably 0,05mm to 0,5mm, more preferably 0,1mm to 0,3mm.

Preferably, the cutting edge of all mentioned blade elements is curved and is oriented so that it is cutting when rotated with its concave side facing forward.

Preferably, the cutting edge of at least one blade elements intersects the equator or extends over the equator of the hemispherical portion.

Preferably, the cutting edge of at least one blade element intersects the apex of the hemispherical portion. The term "intersects the apex" also includes that the respective cutting edge can have a small distance from the apex, preferably of 3 mm or less, more preferably of 2 mm or less. Preferably this means: an imaginary circle is defined by the tip of the cutting edge, wherein the rotation axis of the reamer intersects this circle or is positioned outside this circle; preferably wherein the smallest distance between the rotation axis and the circle is 3 mm or less, more preferably of 2 mm or less.

Preferably, the reamer comprises an apex-blade portion, as a separate blade or a protruding part of at least one of the blade elements, wherein the apex-blade portion intersects with the rotation axes.

Preferably, the cutting edge has one or more chip breakers locally protruding from the cutting section to form a groove in the bone; preferable, wherein the at least one chip breaker has a width of at most 5mm, preferably at most 2mm.

As the bone chips are formed continuously along the blade element(s), they might have a large width. This can have a negative effect on the transportation and storage of the bone chips inside the hollow cavity of the reamer as wider bone chips tend to glut the recess easier. To break the chips in pieces of smaller width, the chip breakers might be added. A chip breaker might be formed by locally elevating the blade's cutting edge so that it cuts deeper into the bone at this location and in result leaving a groove in the cut bone. The groove then acts as breaking point for a bone chip being formed in the following rotation of the reamer. As the groove is small compared to the surface of the cut sphere seating of the cup is not negatively influenced. Chip breakers might e.g. be formed by a pincer. The local blade elevation also generates local peak mechanical stress which supports easier penetration of the cylindrical blade into the bone material and hence result in a lower cutting resistance.

Preferably, the cutting edge of all mentioned blade elements extends over the entire cutting section.

Preferably, the connector portion is integrally formed with the hemispherical portion or is attached to the hemispherical portion.

Preferably, the connector portion comprises at least one bar perpendicular to the equator of the hemispherical portion for attachment of the rotary drill.

Preferably, when not using the movable flap, the body comprises one bridge or more bridges at the recess to connect rigid the portions of the body on both sides of the recess. Preferably, wherein a bridge-blade portion, as a separate blade or a protruding part of the blade element, is positioned at each bridge. The bridge-blade portion can cut any material introduced into the recess and prevent glutting at the location of the bridge.

Preferably, the reamer comprises a reinforcement structure on the concave side of the hemispherical portion. Preferably, wherein the reinforcement structure is integrally formed with the hemispherical portion or attached to the hemispherical portion. Preferably the reinforcement structure stiffens the section of the hemispherical portion on the concave side of the curved recess and/or bridges the curved recess (preferably when not using the movable flap). The reinforcement structure might be designed as a separate part to simplify the molding tool when the body is manufactured by injection molding and which is connected to the reamer body by e.g. ultrasonic welding or an adhesive.

### (4) Drilling machine:

The invention comprise an orthopeadic drilling machine comprising: (i) A shaft connectable to a connector portion of an orthopeadic reamer, preferably one of the above mentioned reamers having the adjustment mechanism. (ii) A main drive for rotating the shaft. (iii) A control element, preferably as a pin in the shaft or a sleeve around the shaft, wherein the control element is adapted to transmit a control movement to the reamer by linear movement and/or rotational movement of the control element relative to the shaft. (iv) And an auxiliary drive for linear moving and/or rotating the control element.

Preferably, the drilling machine comprises a camshaft element, which is actuated by the auxiliary drive for linear moving the control element; either directly or via any transmission element(s).

Preferably, the drilling machine comprises a worm gear rotated by the auxiliary drive, wherein the worm gear rotates the camshaft element.

Preferably, the drilling machine comprises a rotation position sensor, wherein the auxiliary drive is controllable in dependency of a signal form the rotation position sensor.

### (5) Arrangement for milling

The invention comprises an arrangement for milling a hemispherical cavity in a bone, comprising: (i) The orthopeadic drilling machine according to point (4). (ii) An orthopeadic reamer, preferably one of the above-mentioned reamers having the adjustment mechanism adapted to adjust the cutting depth by the control movement of the control element driven by the auxiliary drive of the drilling machine. (iii) A controller arrangement adapted: (A) to determine or receive at least one angle range, which is less than 360° and higher than 0°, (B) to determine or receive the actual rotational angle of the reamer during milling, (C) and to control the auxiliary drive to reduce the cutting depth during the reamer rotates through (within) the angle range, wherein the cutting depth in the angel range is reduced compared to a cutting depth during rotation outside the angle range.

The controller arrangement can comprise one computational unit or several connected computational units. The controller arrangement can, at least parts of the controller, be integrated in the drilling machine.

Preferably, the controller arrangement is adapted to monitor the orientation of the bone and to control the auxiliary drive in dependency of the angle range and the orientation of the bone.

Preferably, the controller arrangement is adapted to monitor the orientation of the drilling machine and to control the auxiliary drive in dependency of the angle range and the orientation of the drilling machine.

Preferably, the controller arrangement is adapted to determine or receive the initial rotational angle of the reamer before the main drive rotates the reamer and to keep track of the actual rotational angle of the reamer by receiving a signal from a rotation position sensor within the drilling machine.

The initial rotation angle can e.g. be determined in that the reamer can only be attached to the drill in one orientation, or by a user input, or by a registration step performed with a surgical navigation system.

The orientation of the drilling machine, of the reamer, and of the bone (e.g. the hip) can be tracked in 3D space using a tracking system such as a surgical navigation system. The rotational position (rotational angle) of the reamer around the reamer rotation axis can be measured by the rotation position sensor. Combination of the navigation and position sensor data allows the controller to calculate the exact pose of the reamer in 3D space and in relation to the bone. Critical areas (angle range) of the bone where less or no bone shall be removed can be defined by the user, e.g using a 3D preoperative surgery plan. A target control element position is calculated for each rotation position of the reamer during it's revolution so that whenever the cutting edge would cut bone of one of the defined critical areas the control element is moved, e.g. pushed out, to transmit the control movement to the control arrangement of the adjustment mechanism of the reamer, so that cutting is reduced or prevented by one of the adjustment mechanisms described above. Once the cutting edge passes the critical area the control element is moved back to its initial position and the reamer is cutting with the maximum cutting depth. Repeating this operation for each revolution of the reamer allows for selective cutting of the bone.

Provided that a desired end position of the reamer in respect to the patient's bone is further given as input to the controller a desired reamer path can be computed. During the reaming process the current position of the reamer is compared to the desired position on the calculated target reamer path resulting in a displacement vector.

The control element movement is then controlled in such a way that cutting is only performed in a rotation sector outside the defined angle range. The center of the rotation sector is roughly located in direction of the displacement vector. Repeating this operation for each revolution of the reamer allows for directional reaming.

### (6) Methods

The invention comprise a method for manufacturing an orthopeadic reamer according to point (1) or (2), preferably including any optional feature disclosed under point (1) or (2). Optional feature(s) of the method are as follows:
Preferably, the method comprises following step: Forming the hemispherical portion out of metal; preferably by forming a metal sheet.

Thereby, preferably, the method comprises following step: Forming the curved recess by punching, and/or 3D-laser cutting, and/or CNC machining. In addition or alternatively, preferably, the method comprises following step: Forming the connector portion as separate part and attaching it to the hemispherical portion.

When manufacturing the reamer according to point (1) and the hemispherical portion out of metal, preferably, the method comprises following step: Attaching the blade element to the hemispherical portion by welding and/or soldering and/or snap fitting and/or press fitting.

When manufacturing the reamer according to point (1) and not manufacturing the hemispherical portion out of metal, preferably, the method comprises following step: forming the hemispherical portion of plastic. Preferably, the method comprises following step: Forming the hemispherical portion by injection molding or 3D-printing. Preferably, the method comprises following step: Forming the connector portion as separate part and attaching it to the hemispherical portion.

When manufacturing the reamer according to point (1) and the hemispherical portion out of plastic, preferably, the method comprises following step: Attaching the blade element to the hemispherical portion by gluing and/or snap fitting and/or press fitting and/or welding, or by overmolding by the injection molded hemispherical portion or overprinting by the 3D-printed hemispherical portion. If the blade element is welded on, it is preferably fixed by ultrasonic welding or hot air welding, whereby the plastic of the hemispherical portion is melted near the blade element in order to fix the blade element.

When manufacturing the reamer according to point (1), preferably, the method comprises following step: Manufacturing the blade element by cutting a ring from a metal tube. The cut ring can preferably be machined by cutting and/or milling and/or grinding. Cutting the blade element from a metal tube limits production costs and waste material significantly. Instead of cutting the blade element from a tube, the blade element can also be milled or lathed from a body.

Further details, advantages and features of the present invention are shown in the following description of embodiments based on the drawing. The following description and embodiments, as well as the figures are exemplary in nature and are not intended to limit the scope, applicability, or configuration of the invention.
- Fig. 1: the inventive reamer according to all embodiments positioned at a bone,
- Fig. 2 to 7: details of the inventive reamer having an embedded ring-shaped blade element,
- Fig. 8 and 9: details of the inventive reamer having two blade elements,
- Fig. 10 to 15: details of the inventive reamer having a metal body,
- Fig. 16 and 17: details of the inventive reamer having a plastic body,
- Fig. 18 and 19: details of the inventive reamer having an integral curved blade element,
- Fig. 20 to 26: details of the inventive reamer having an adjustment mechanism,
- Fig. 27 and 28: details of the inventive drilling machine and the inventive arrangement, and
- Fig. 29 and 30: further details of the inventive reamer according to all embodiments.

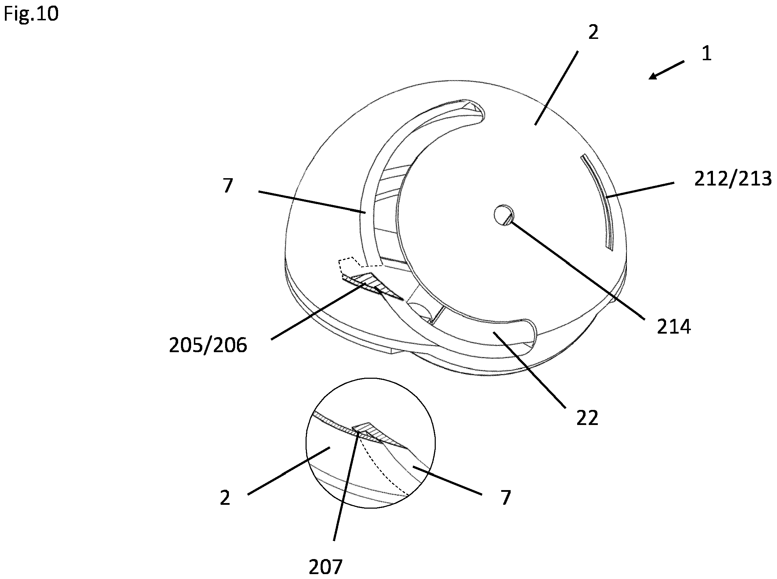

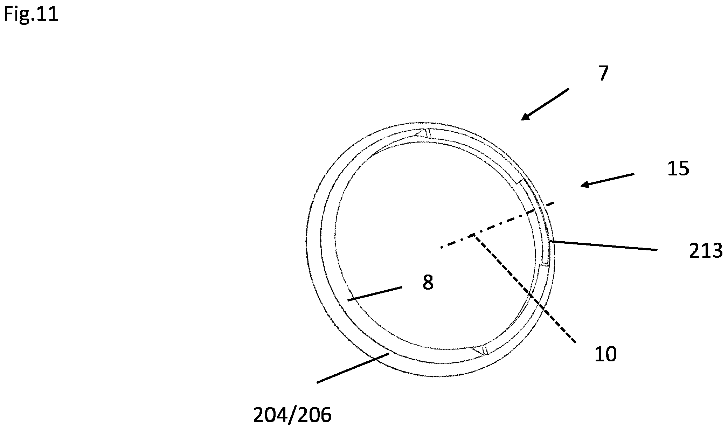

The inventive orthopeadic reamers 1, the inventive methods for manufacturing the orthopeadic reamers 1, the inventive orthopeadic drilling machine 700, and the inventive arrangement 800 for milling a hemispherical cavity in a bone 900 are explained in more detail below. The methods for manufacturing are e.g. disclosed by the detailed description of Fig. 10 to 19.

Unless otherwise stated, reference is always made to all figures.

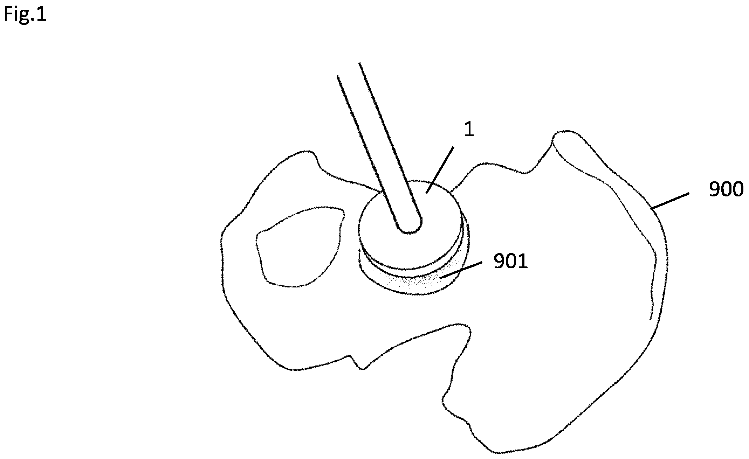

The Figures disclose an orthopeadic reamer 1, preferably acetabular reamer, for milling a hemispherical cavity 901 in a bone 900 (see Fig. 1), preferably adapted for single use, comprising a body 2, having a hemispherical portion 3 spanning a cavity 4, preferably to store cut bone debris, and having a connector portion 5 adapted for attachment of a drilling machine 700 to rotate the reamer 1 around a rotation axis 6, and comprising at least one blade element 7 having a curved cutting section 8 with a sharp cutting edge 9.

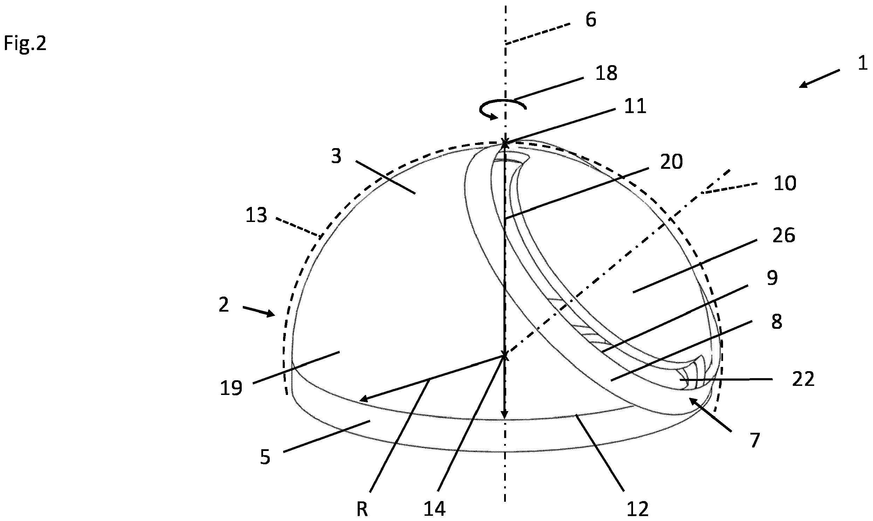

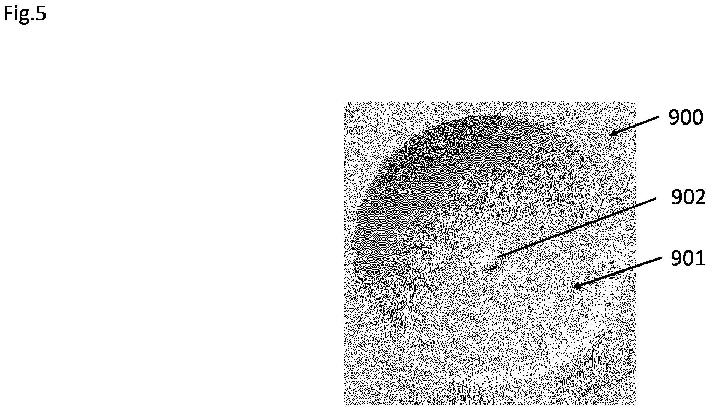

Fig. 2 to 17 show the reamer 1 with the at least one blade element 7 as "ring-shape blade element(s)". The blade element(s) 7 is/are embedded in the hemispherical portion 3.

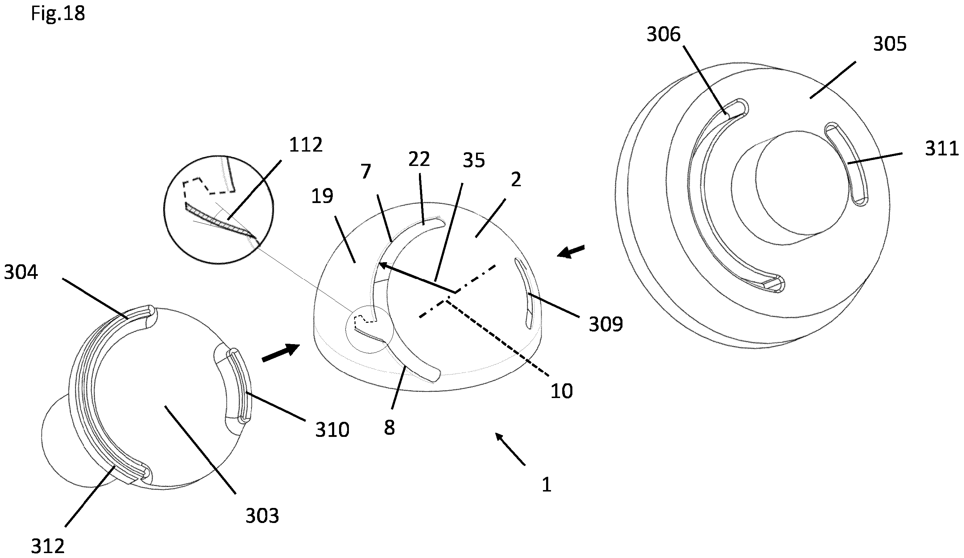

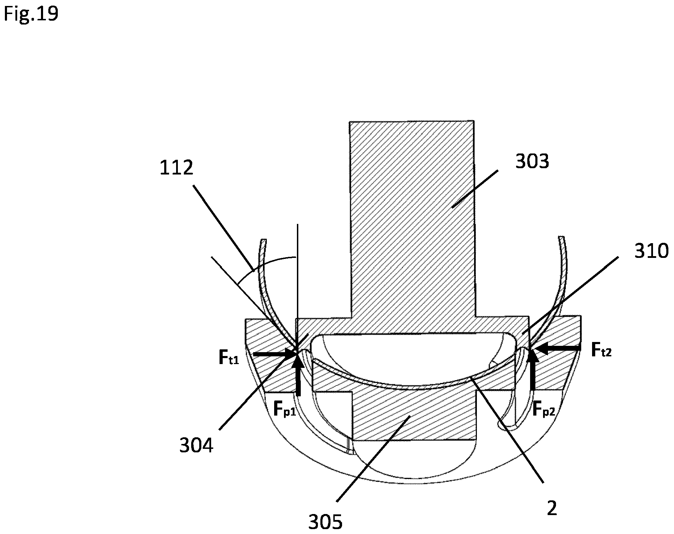

Fig. 18 and 19 show the reamer 1 with the blade element 7 as "curved blade element". The blade element 7 is an integral part of the hemispherical portion 3.

All Figures disclose optional features as explained in the general part of the description, which can be applied for both the reamer with embedded ring-shaped blade elements 7 and the reamer with integral curved blade elements 7.

For all embodiments, a blade axis 10, preferably for each blade element 7, is defined perpendicular to a radius 35 of the blade element 7, and the blade axis 10, preferably of each blade element 7, is inclined to the rotation axis 6.

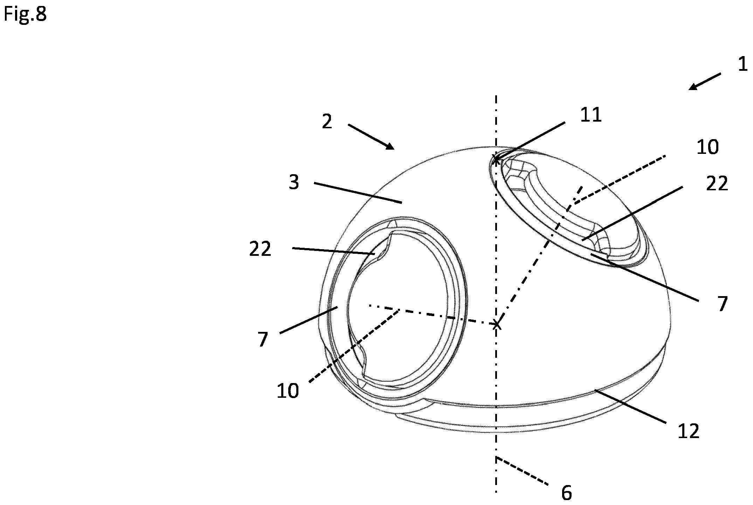

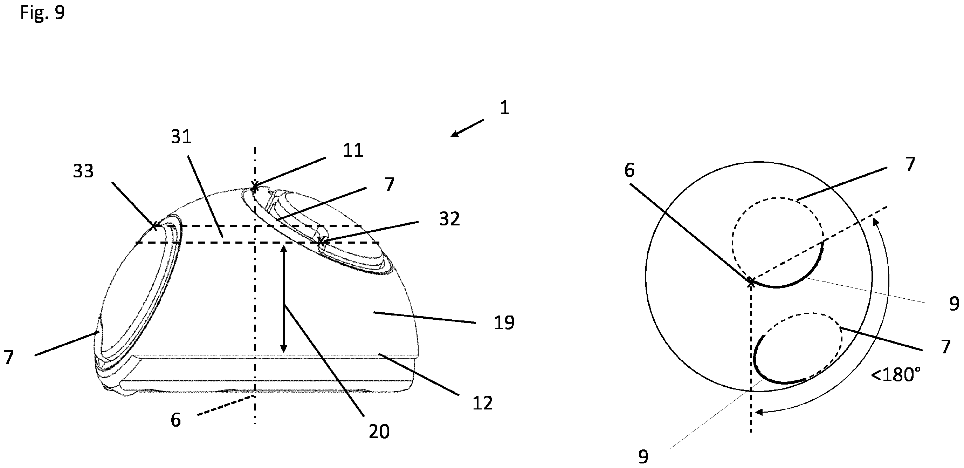

The reamer 1 preferably comprises only one blade element 7. However, there is also an embodiment (Fig. 8 and 9) with more than one blade element 7, so that the features given below with regard to the blade element 7- unless otherwise mentioned - preferably apply to all blade elements 7.

In all embodiments, the connector portion 5 and its connection to the hemispherical portion 3 is rigid, so that the connector portion 5 and the hemispherical portion 3 rotate around the common rotation axis 6 without wobbling relative to each other.

In all embodiments, the hemispherical portion 3 extends from its apex 11 to its equator 12. The connector portion 5 is located in the area of the equator 12. The cavity 4 of the reamer 1 is formed between the inside of the hemispherical portion 3 and the connector portion 5, which is in the area of the equator 12. The hemispherical portion 3 according to the embodiments forms a continuous hemispherical surface over the entire circumference and from the apex 11 to the equator 12 in order to guide and support the reamer relative to the bone. However, a minimally invasive design (not shown) is also possible in which the hemispherical shape is not continuous so that the reamer is narrower and can therefore be inserted into the patient's body through smaller openings.

In all embodiments, the connector portion 5 protrudes from the equator 12 of the hemispherical portion 3. The outer shape of the connector portion 5 is convex, thereby having a smaller diameter than the hemispherical portion 3.

In all embodiments, the rotation axis 6 of the reamer 1 intersects the apex 11 of the hemispherical portion 3 and the center point of the connector portion 5.

In all embodiments, the hemispherical portion 3 has a slit formed curved recess 22 at each cutting section 8. The curved recess 22 is adjacent and in cutting direction directly in front of the cutting edge 9, to transport the cut bone debris into the cavity 4.

In all embodiments, the blade axis 10 is inclined to the rotation axis 6. Thereby, the blade element(s) 7 is/are adapted in size and position, such that the cutting edge 9 of the cutting section 8 moves on a virtual cutting sphere 13 when the reamer 1 is rotated around its rotation axis 6. The blade axis 10 intersects the midpoint 14 of the hemispherical portion 3.

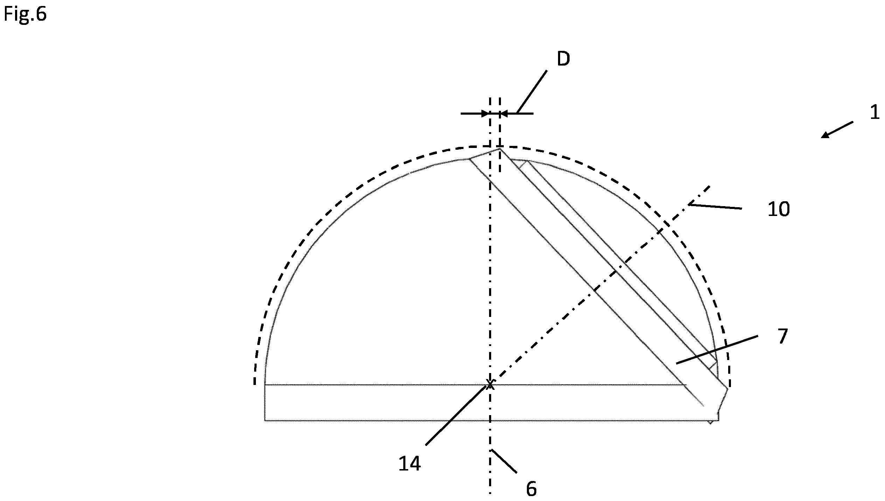

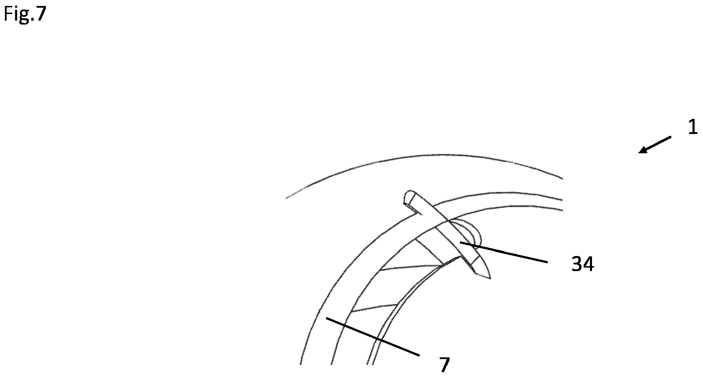

To cut a half sphere (hemispherical cavity 901) in the bone 900, in particular with only one blade element 7, the cutting edge 9 of the blade element 7 should intersect the apex 11 and the equator 12 of the cutting sphere. When using more than one blade elements 7, the cutting edge 9 of one blade element 7 can intersect the apex 11 and the cutting edge 9 of another blade element 7 can intersect the equator 12. The term "intersects the apex" includes that the respective cutting edge 9 can have a small distance D from the apex 11 - which is shown in Fig. 6. Even with this small distance D, an uncut portion 902 at the apex of the cutting sphere can be avoided. Further, according to Fig. 7, the reamer 1 can comprise an apex-blade portion 34, as a separate blade or a protruding part of the blade element 7, wherein the apex-blade portion intersects with the rotation axes 6. The distance D and the apex-blade portion 34 are shown for one embodiment but can be used for all embodiments of the reamer 1.

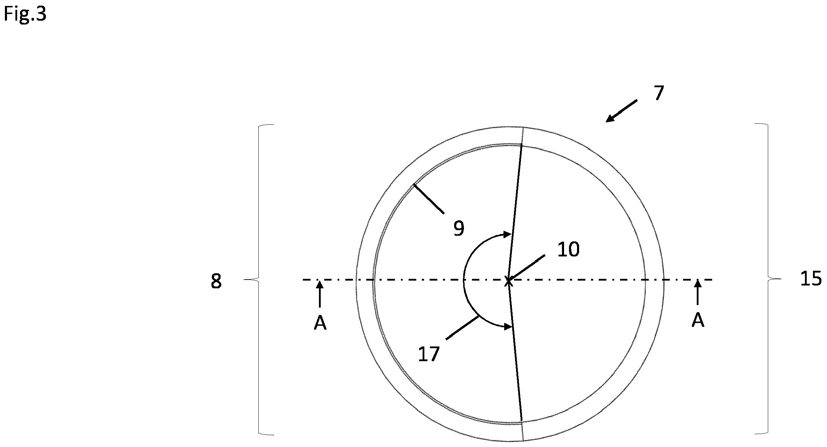

According to all embodiments, except for Fig. 18 and 19, the at least one blade element 7 is a closed ring extending over a ring angle of 360° around the blade axis 10 (see e.g. Fig. 3, 4, 11). In a not shown embodiment, the blade element 7 is an open ring extending over a ring angle of at least 200°, preferably at least 250°, around the blade axis 10. The ring angel is defined around the blade axis 10.

According to all embodiments, except for Fig. 18 and 19, the at least one blade element 7 is circular but can also be elliptical (not shown).

According to all embodiments, except for Fig. 18 and 19, the cutting section 8 of the at least one blade element 7 merges into a support section 15 (see Fig. 3) of the blade element 7, so that the circumference of the blade element 7 is formed by the cutting section 8 and by the support section 15.

According to all embodiments, except for Fig. 18 and 19, the support section 15 can be so deeply embedded in the body 2 that the body sections close to the support section 15 contact the bone 900, wherein the support section 15 preferably does not contact the bone 900.

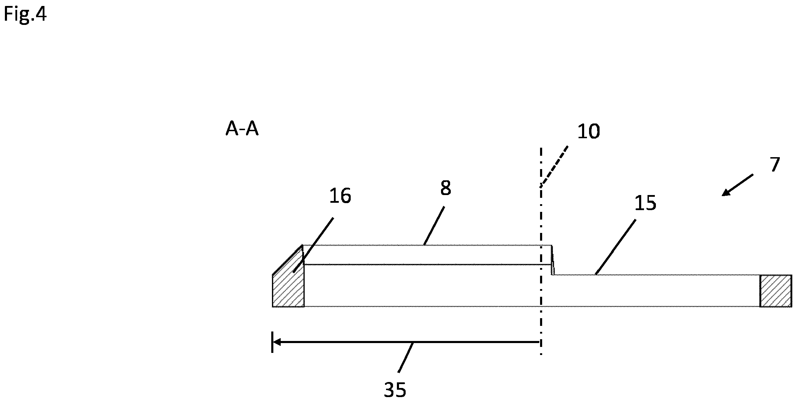

According to all embodiment, except for Fig. 18 and 19, the area of the cross section 16 (see Fig. 4) of the blade element 7 can be at most 100 qmm (square millimeter), preferably at most 50 qmm; wherein the area is measured at the thickest point of the cutting section 8, which is preferably the thickest point of the blade element 7. The area of the cross section 16 is considered in a plane A-A (see. Fig. 4) in which the blade axis 10 lies. The plane intersects the blade element 7 twice, but only one of the two intersections count as "area of the cross section 16".

According to Fig. 3, the cutting section 8 extends over a section angle 17 of little more than 180° around the blade axis 10. Although different sections are defined on the blade element 7, the blade element 7 is a one-piece component. The support section 15 has no cutting edge and is preferably positioned as to have no contact to the bone 900. The support section 15 supports the stiffness of the blade element 7 and the attachment of the blade element 7 to the body 2.

When using the reamer 1, it is rotated (direction of rotation 18) so that the support section 15 is at the front and the cutting section 8 is at the rear in the direction of rotation 18. This is why the support section 15 can also be referred to as leading section and the cutting section 8 as non-leading section. For the same reason, the cutting edge 9 has a leading edge or side and a non-leading (also called trailing) edge or side. As the blade element 7 is ring-shaped, the cutting section 8 and the support section 15 are curved. Consequently, the cutting section 8 has a concave side that points forwards in the direction of rotation 18 when the reamer 1 rotates. The cutting edge 9 is aligned in such a way that it cuts.

With exception of Fig. 8 and 9, the reamer 1 of all embodiments is designed as one blade reamer having only the one blade element 7. Thereby, the reamer 1 has no other cutting elements and/or no other grinding elements.

Alternatively, according to Fig. 8 and 9, the reamer 1 is designed as two blade reamer having only two of the blade elements 7. Thereby, the reamer 1 has no other cutting elements and/or no other grinding elements. According to a not shown embodiment, the reamer is designed as three or four blade reamer having only three of the ring-shaped blade elements, and has no other cutting elements and/or no other grinding elements. The two blade design is shown for one embodiment but can be used for all embodiments of the reamer 1.

According to Fig. 8 and 9, the reamer 1 can have two blade elements 7; the top blade element 7 being closer to the apex 11 as the bottom blade element 7. The blade elements 7 have a diameter which makes the blade elements 7 extend over a portion of the latitude between the reamer apex 11 and equator 12. The blade elements 7 are positioned along the latitude as such that their combined blade portions together extend from the reamer apex 11 beyond the reamer equator 12. Further the blade elements 7 are positioned as such that their cutting sections 8 have an intersection portion 31 - a section of the latitude where a section of both blade elements 7 is placed. The intersection portion 31 is advantage as the cutting force of the top blade's bottom point 32 and the bottom blade's top point 33 becomes zero as the angle of attack is approaching zero. The cutting sections of the two blade elements 7 might be separated by an angle of less than 180°, preferably 130° around the reamer rotation axis 6 from each other, so that surface of hemispherical portion is in contact with bone during cutting - as can be seen in the right picture of Fig. 9.

Besides the intersection portion 31 and particular at the area close to the reamer equator 12 the reamer has two free areas being opposed to the blade elements 7 where the outer surface of the reamer body 2 is sliding on the bone 900. The area close to the equator 12 is referred to as single cutting area 19. The blade axis 10 of all blade elements 7 are inclined to each other and are inclined to the rotation axis 6. The two blade elements 7 do not intersect with each other. When using the reamer 1, however, the two blade elements 7 partially cut the same area of the bone 900. The curved recess 22 is provided at each of the two blade elements 7. The two blade elements 7 are arranged next to each other and not inside each other. Each blade element 7 is designed as a closed ring.

Shown in Fig. 2 and 9, but applicable to all embodiments, a single cutting area 19 is defined on the hemispherical portion 3 of the body 2, wherein only one blade element 7 is disposed in the single cutting area - even if the reamer 1 comprises two or three or four of the blade elements 7. No other cutting elements and/or no grinding elements are disposed in the single cutting area 19.

The single cutting area 19 is a full circumference ring area that extends from the equator 12 of the hemispherical portion 3 in direction of the apex 11. A height 20 (see Fig. 9) of the single cutting area 19 is measured from the equator 12 in direction of the apex 11 parallel to the rotation axis 6 of the reamer 1. The height 20 is in the embodiment with one blade element 7 equal or approximately equal to R, and in the embodiment with two blade elements 7 approximately 0,7*R, with R being the radius of the hemispherical portion 3. The single cutting area 19 is designed as a smooth, spherical surface to support and guide the reamer 1 gliding on the bone 900.

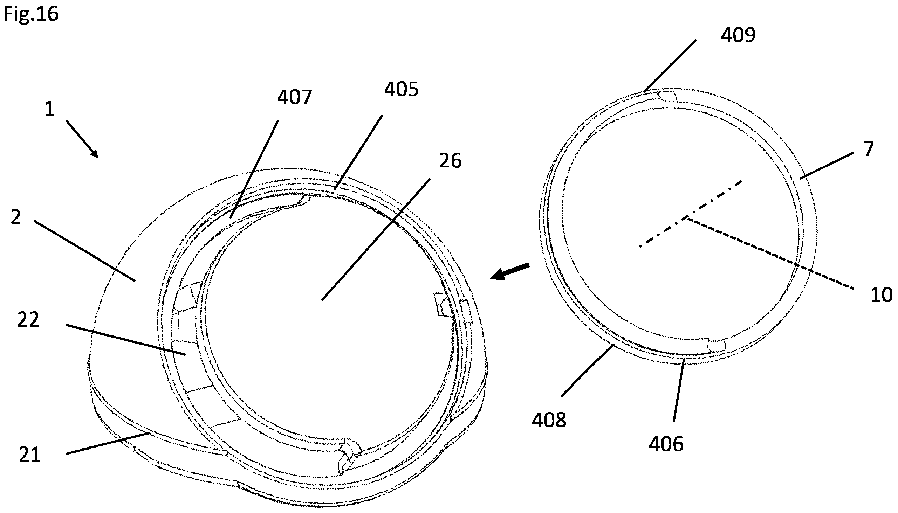

The reamer 1 of all embodiments, preferably with a body made of plastic, can comprise an X-ray absorber 21 (shown in Fig. 16) in the body 2. The X-ray absorber 21 can be a part, in particular an elliptic ring, or the X-ray absorber 21 can be made by an X-ray absorbing material added to the plastic compound of the body 2.

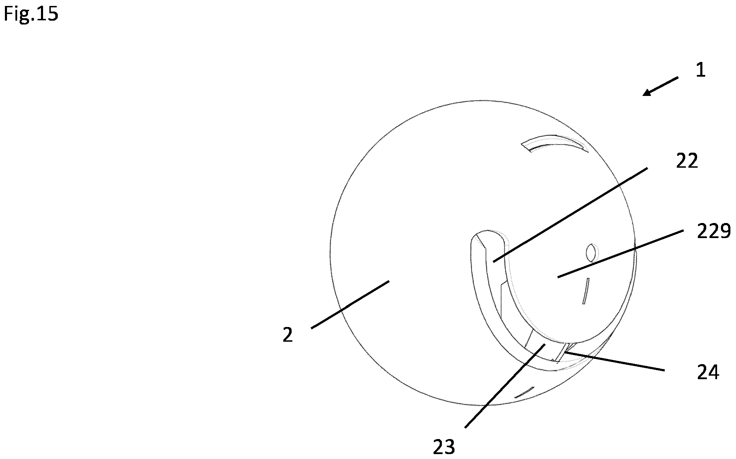

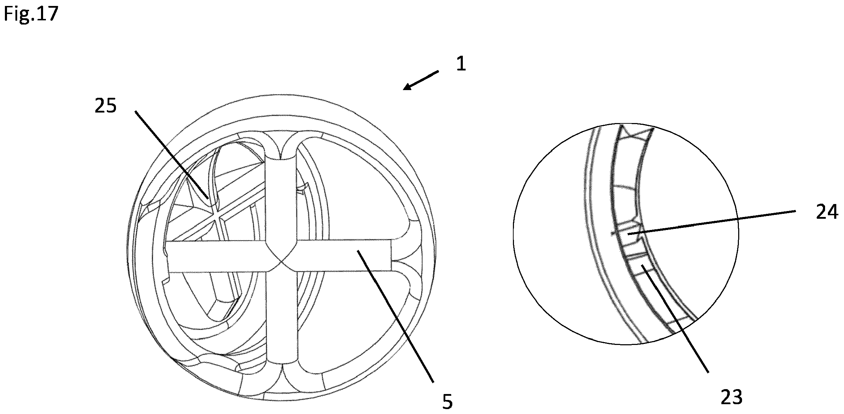

According to all embodiments, the body 2 can comprise one bridge 23 or more bridges 23 at the recess 22 to connect the sections of the body 2 on both sides of the recess 22. This is e.g. shown in Fig. 15 and Fig. 17. A bridge-blade portion 24, as a separate blade or a protruding part of the blade element 7, can be positioned at each bridge 23 as shown in Fig. 17. Further, the reamer 1 can comprise a reinforcement structure 25 on the concave side of the hemispherical portion 3. This is e.g. shown in Fig. 17.

### Reamer with metal body

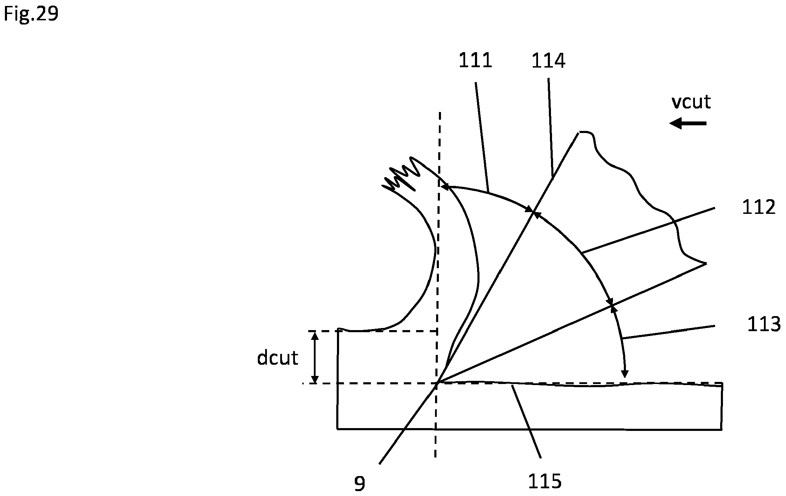

According to Fig.10 to 15, the reamer body 2 is formed out of a metal sheet which is bend by metal spinning or other metal forming techniques into a hollow hemisphere of a defined diameter. Metal spinning has the advantage of comparably low tooling costs and tight tolerance control of the outside and inside surface of the created hollow hemisphere. The blade element 7 might be formed from metal rods or preferably metal tubes using preferably a continuous CNC driven lathe or milling machine. The blade element 7 has the cutting section 8 with a blade angle 112 (see Fig. 29) of approx. 30° forming the cutting edge 9 of the blade element 7 generating a sufficient clearance angle 113 for the blade element 7 to cut. The edges defining the blade angle 112 might be additionally grinded to remove any remaining uneven parts of the cutting edge 9 and hence create a very sharp edge.

The blade element 7 further comprises a connection portion 204 which is intended to mate with an inner surface 205 of the reamer body 2. The connection portion 204 therefore comprises at least one connecting surface 206 being spherically shaped with the same radius as the inner surface 205 of the reamer body 2 creating a surface-to-surface contact when mated.

Alternatively, as shown in the small detail view of Fig. 10, the blade element 7 comprises at least one circular shaped edge 207 creating a line contact with the reamer body's inner surface 205 when mated. When assembled as such the cutting edge 9 is protruding the reamer body 2.

The curved recess 22 in the reamer body 2 as well as a notch for bone removal might be formed by laser cutting, stamping or their like. As described above only the cutting section 8 of the blade element 7 cuts bone. Hence there is no need for the leading edge (support section 15) to protrude the reamer body 2. Hence the cutting portion of the leading edge (support section 15) is removed leaving only the connection portion 204.

The reamer body 2 and blade element 7 are assembled using a clamping system and are then connected by e.g. laser welding or an adhesive. Depending on how tight manufacturing tolerances of the blade element 7 and reamer body 2 can be controlled two different types of clamping mechanism are required.

When manufacturing tolerances are sufficiently small the blade element 7 can be aligned solely by the features of the reamer body 2. Hence the clamping mechanism only needs to hold the aligned mating partners in place. Such alignment can e.g. be achieved the following. The correct radial positioning is achieved by mating the reamer body's inner surface 205 with the connecting surface 206 of the blade element 7 as described above. The orientation of the blade axis 10 is constrained by mating the cylindrical shaped faces of the recess 22 and a cylindrical face of the connection portion 204 of the blade element 7. The remaining rotation around the blade axis 10 might be constrained by an additional opening 212 in the reamer body 2. For mating with this additional opening 212 an element 213 of the connecting portion at the support section 15 of the blade element 7 is elevated so that it engages with the additional opening 212.

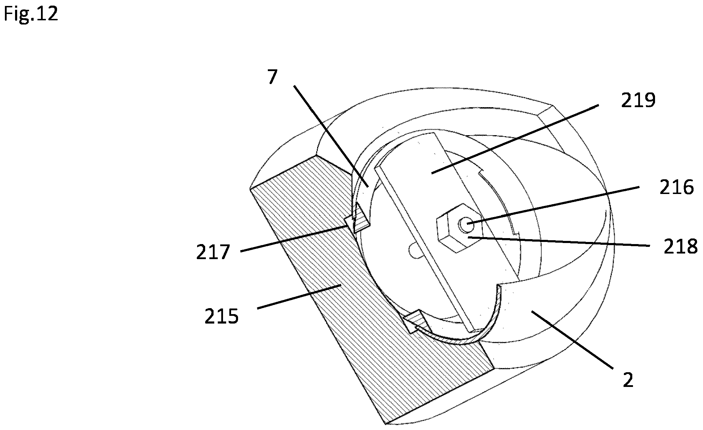

According to Fig. 12, the clamping system 215 might for example be a metal part with a spherical cavity which fits the reamer body 2. It comprises a centring rod 216 which connects through a hole 214 in the reamer body 2 and aligns the reamer body 2 with the clamping system 215. Further the clamping system comprises a groove 217 where the blade element 7 penetrates the reamer body 2 so that there is no contact between the blade element 7 and the clamping system 215. The clamping system 215 might either be formed from magnetic material to keep the reamer body 2 and blade element 7 in place by magnetic forces or the centring rod 216 further comprises a threat which allows for attachment of a tensioning screw 218 and pressure plate 219 which holds the blade element 7 and reamer body 2 in place by compressive forces during assembly.

When manufacturing tolerances are too wide for accurate alignment of the components a clamping device can be uses, which not only holds the mating partners in place but also defines their relative position and orientation to each other. Such a clamping device might be similar in form and shape to the clamping system 215 described beforehand however the groove 217 below the cutting edge of the blade element 7 is formed as such that when the cutting edge 9 of the blade element 7 is positioned within the groove 217 its radial positioning in respect to the outer surface of the reamer body 2 is defined. Therefore, the groove might have a planar connection surface or a connection surface of spherical shape. The blade element connection portion 204 on the other hand is formed as such that when correct radial alignment is provided by the clamping mechanism a gap is present between the blade element' connecting surface 206 and inner surface 205 of the reamer body 2. The gap between the two parts is then closed by the joining process (welding, gluing, or their like).

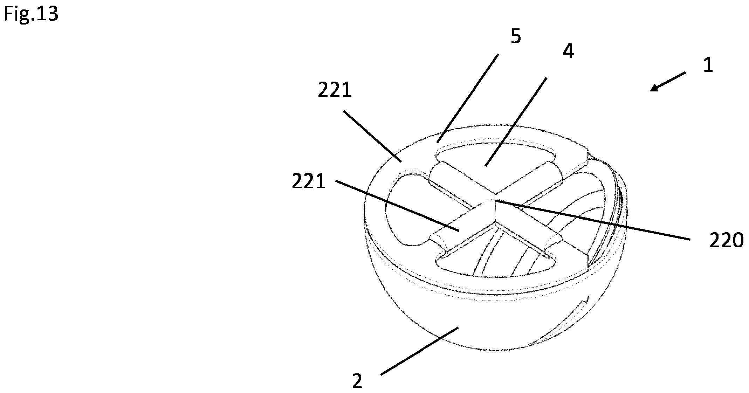

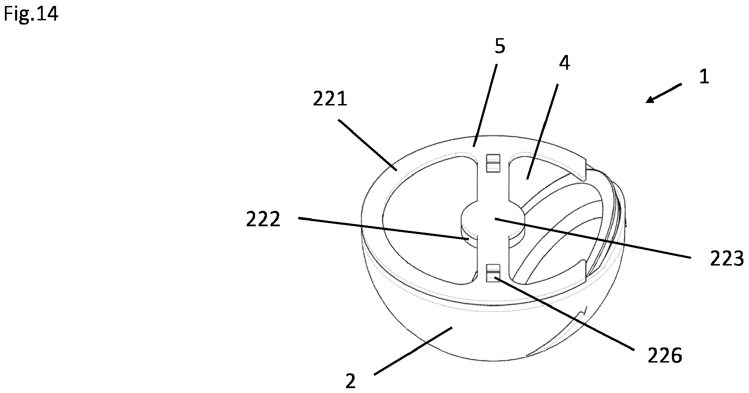

According to Fig. 13 and 14, the reamer body 2 is further joined with the connector portion 5 which allows for attachment of the drilling machine 700 providing the torque for the reaming. Common connector shapes used in reaming systems are the 'crossbar' and the 'bridgeback' design. The 'crossbar' design 220 might e.g. be formed from flat sheet metal 221 which is cut by laser cutting, stamping or their like. The cut shape comprises two bridges intersecting at 90° with a width of approximately 4mm which are enclosed by a cylindrical of similar thickness at which the connector portion 5 is joined to the reamer body 2 by e.g. welding or an adhesive. Additionally, two plastic parts 222 are joined to the top and bottom of the metal sheet e.g. by ultrasonic welding or an adhesive. When joined the components form two intersecting cylindrical rods with a diameter of approximately 5mm.

The 'bridgeback' design 223 according to Fig. 14 might e.g. be formed from flat sheet metal 221 which is cut by laser cutting, stamping or their like. The cut shape comprises one bridge with a width of approximately 6mm which comprises a circular shape at the reamer centre. The bridge is further enclosed by a metal ring where the connector portion 5 is joined to the reamer body 2 by e.g. welding or an adhesive. Additionally, a plastic part 222 is joined either to the top or bottom of the metal sheet e.g. by an adhesive or a clip 226. When joined the components form a bridge of a thickness of approximately 3mm in the commonly known 'bridgeback' connector shape. The two connector designs might also be formed solely from plastic material or any other material. A plastic only design has the advantage that the bridge section of the connector can be separated with a clipper to create a larger opening and simplify removal of the cut bone material stored in the hollow of the reamer 1. Sufficient removal of the cut material is important for waste disposal of the used reamer 1.

According to Fig. 15, the reamer 1 might further be equipped with a reinforcing bridge 23 stiffening the portion 229 of the body on the concave side of the recess 22. The portion 229 being enclosed by the recess 22. The bridge 23 might e.g. be formed from sheet metal and reaches from one side of the recess 22 to the other. The so formed bridge 23 adds sufficient mechanical support for the portion 229 and prevents bending during the reaming proceed which would negatively affect the cutting depth of the reamer 1. The bridge 23 can be equipped with a bridge-blade portion 24 which cuts any material introduced into the recess 22 during the ream which would otherwise collect at the bridge 23 and thereby glut the reamer 1.

### Reamer with plastic body

Fig. 16 and 17 show the reamer body 2 formed out of plastic by injection molding, or 3D printing or milling or their like. The reamer body 2 comprises the recess 22 to transport the cut bone into the inner portion of the reamer body 2 and a mating feature for the blade element 7 to be attached. The mating feature is sunk into the outer surface of the reamer 1 and defines the pose of the blade element 7 when assembled. The mating feature comprises a first surface of cylindrical shape 405 which connects to the outside surface 406 of the blade element 7. Further it comprises a flat surface 407 which defines the depth with which the blade element 7 might be sunk into the reamer 1 and which mates with a bottom surface 408 of the blade element 7. The blade element 7 might be rigidly attached to the body 2 by means of a press fit where the outer surface 406 of the blade element 7 has the same or larger diameter than the first surface of cylindrical shape 405 of the body 2 and is pressed into the mating feature during assembly. Alternatively or additionally the blade element 7 might be fixed using an adhesive substance. When mated as such the reamer body 2 and blade element 7 form a rigid connection. For correct alignment the blade element 7 might have a circulating overhang 409 formed at its outside portion which allows an assembly tool to hold the blade element 7 in a desired position and apply sufficient pressure to insert blade element 7 into the reamer body 2. Additionally, one or multiple alignment features 411 to simplify the alignment of the blade element 7 might be molded into the reamer body 2.

It is common practice during surgery to check correct position and orientation of the reamer 1 on a fluoroscopic image. As the reamer 1 is manufactured from plastic its visibility within the fluoroscopic image is limited. To allow better judgement a ring as X-ray absorber 21 of a material well visible in X-ray images such as metal might be added at the reamer equator. Depending on the orientation of the reamer 1 to the fluoroscopic device the ring is shaped as an ellipse whose shape allows the surgeon to judge the orientation of the reamer 1 in respect to the patient's anatomy. Alternatively, a contrast medium might be added to the plastic compound to increase visibility of the whole reamer.

The reamer body 2 is further connected to a connector portion 5 being of one of the designs described above or is manufactured as one part. The outer shape might also be of the spherical shape of the reamer body 2. This is different to conventional reamers which have an outer surface of cylindrical shape below the reamer equator which is a necessity of the manufacturing process. A continued spherical shape has the advantage that the reamer 1 can be rotated freely within the prepared joint without the cylindrical shape to leaver the reamer 1 out of the joint socket. When assembled as such the reamer functions as described above. The reamer body might additionally have a reinforcement structure 25 formed on the inside surface of the body 2 to stiffen the part enclosed by the recess 22. The reinforcement structure 25 might be designed as a separate part to simplify the molding tool when the reamer 1 is manufactured by injection molding and which is connected to the reamer body 2 by e.g. ultrasonic welding or an adhesive. Further, as shown in Fig. 17, a material bridge 23 crossing the recess 22 might be added which can further be equipped with a bridge-blade portion 24 to cut any material introduced into the recess 22 and prevent glutting at the location of the bridge 23.

### Reamer with integral curved blade element

Fig. 18 and 19 show the orthopeadic reamer 1 by a stamping and bending action preferably in one process, comprising the body 2, having a hemispherical portion 3 spanning the cavity 4 to store cut bone debris, and having the connector portion 5 adapted for attachment of the drilling machine 700 to rotate the reamer 1 around the rotation axis 6, and comprising at least one, preferably only one, two or three, of the curved blade element(s) 7 with cutting section 8 and the sharp cutting edge 9 in the hemispherical portion 3. The hemispherical portion 3 and the at least one curved blade element 7 is one part formed of metal. The at least one curved blade element 7 is formed by the recess 22 punched in the hemispherical body 2. Thereby the cutting edge 9 is elevated above the surface of the hemisphere by the desired cutting depth. The reamer has the single cutting area 19 as explained above. For a multi-blade version all cutting edges 9 are arranged in a section of the hemisphere smaller 180°.

According to Fig. 18 and 19, the reamer 1 including body 2 and the curved blade element 7 (trailing edge) is formed by a stamping and bending action, preferably in one process, with forming the curved recess 22 for transportation of bone debris into the reamer cavity 4. A cutting / stamping tool 303 with a cutting element 304 of the shape of the recess 22 might therefore be pressed against the inner surface of the reamer body 2 while a matrix component 305 having a matrix component recess 306 in the shape of the recess 22 is pressed against the outside surface of the reamer 1. A pressing force is then applied resulting in the cutting/stamping tool 303 to penetrate the reamer body's 2 wall and thereby forming the recess 22 and gliding into the matrix component recess 306. The stamped recess 22 has the shape of approximately a half cylindrical.

As the stamping direction is at an angle to the radial direction of the reamer 1 at any given point on the cutting section 8 a sharp blade angle 112 is formed. When the cutting element 304 is pressed against the inside surface of the reamer 1 it will experience pressure forces Fₚ₁ oriented in the direction of the stamping action but also transverse forces Fₜ₁ oriented in orthogonal direction due to the spherical shape of the inner surface. These transverse forces Fₜ₁ create a momentum acting on the cutting/stamping tool 303. To prevent an excessive momentum acting on the cutting/stamping tool 303 a second opening 309 might be formed by a second cutting element 310 and second matrix component recess 311. The pressure force Fₚ₂ and transverse forces Fₜ₂ acting at the second cutting element 310 are counteracting the momentum generate by the forces Fₚ₁ and Fₜ₁.

It is known from metal stamping that the cutting/stamping tool 303 and matrix component 305 can be designed as such that the edge of the formed recess 22 is sharp and slightly elevated, often referred to as stamping burr. The elevation is created as plastic deformation of the cut material in the movement direction of the cutting/stamping tool 303 is occurring. The deformation can e.g. be controlled by the gap between the cutting element 304 and matrix component recess 306. The elevation of the curved blade element 7of the recess 22 is further increased by a conical surface 312 at the outside portion of the cutting element 304 which displaces the material at the curved blade element 7 in the direction of movement. This way the curved blade element 7 is elevated further than the other edges. When correctly controlled the elevation of the curved blade element 7 is uniform and of a desired height so that the deformed cutting edge 9 is positioned on a desired spherical surface which is the cutting sphere of the reamer 1. Formed as such and when attached with a connector of one of the described types the reamer 1 functions as described above.

The hemispherical portion 3 according to all embodiments can have a section, referred to as flap 26, of the hemispherical portion 3 on the concave side of the curved recess 22. The flap 26 can be adjustable in order to adjust the cutting depth of the cutting section 8. The flap 26 is the section inside the ring-shaped blade element 7; or inside the curved blade element 7 of the one-part design according to Fig. 18 and 19.

The flap 26 can be designed to be bendable inward by axial force on the reamer to increase the cutting depth in dependency of the axial force. Preferably, whereby the flap 26 - if no axial force is applied - protrudes over the cutting edge 9.

### Reamers equipped with adjustment mechanism

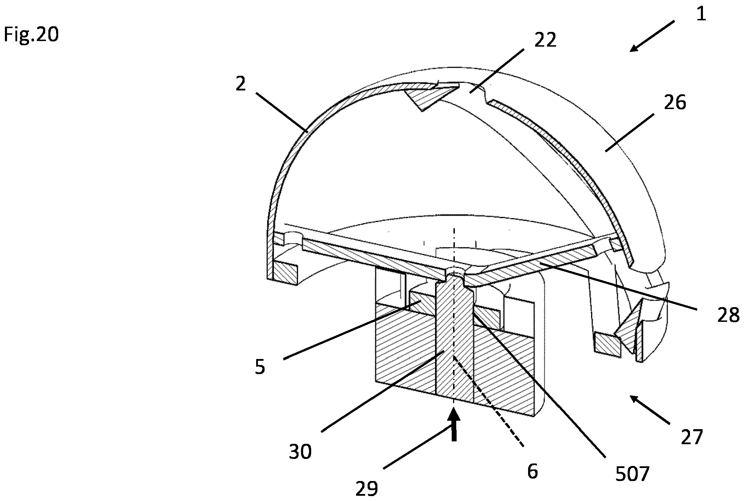

According to Fig. 20 to 25, the reamer 1 of any forgoing embodiment might be equipped with an adjustment mechanism 27 supporting the flap 26, which allows for elastic deformation in a desired way.

The adjustment mechanism 27 is adapted to lift and/or lower the flap 26 relative to the blade element 7 in order to adjust the cutting depth of the blade element 7. Examples of the adjustment mechanism are shown in Fig. 20 to 25.

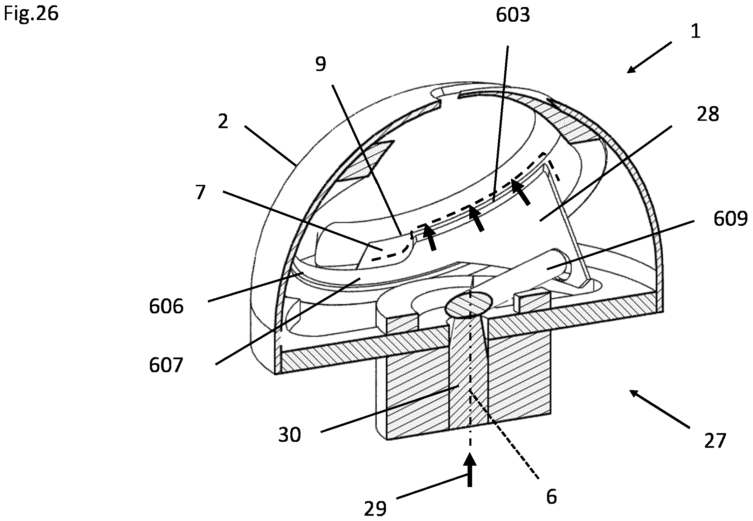

According to Fig. 26, alternatively, the reamer 1 of any forgoing embodiment can have a cut preventing component 28 and the adjustment mechanism 27, wherein the cut preventing component 28 is movable through the recess 22 by the adjustment mechanism 27, so that the cut preventing component 28 can overtop the cutting edge 9 to prevent the reamer 1 from cutting.

The adjustment mechanism 27 has a control arrangement 28, which is adapted to be operated by a control movement 29 of the control element 30 of the drilling machine 700.

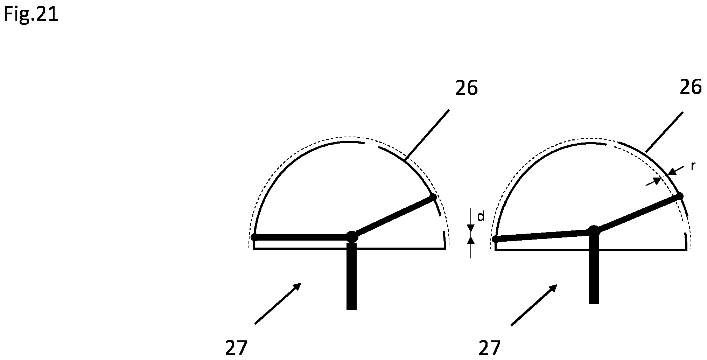

According to Fig. 20 and 21, the adjustment mechanism 27 might be designed in a general V shape connecting the inner surface of the flap 26 to the opposing inner surface of the reamer body 2. These areas are close to the attachment points to the inner surface of the reamer body 2 and the bottom of the V shape. The adjustment mechanism 27 might be of any other profile which provides sufficient rigidity against bending. Areas of flexibility could alternatively be designed as joints.

Further the connector portion 5 of the reamer 1 has a hole 507 in it's centre allowing a control element 30 in form of a pin being introduced into the hollow of the reamer body 2. The tip of the control element 30 is positioned so that it touches the adjustment mechanism 27 at the bottom of the V shape creating a form fit which allows for force transmission. In a first state the control element 30 is positioned in a first position touching the adjustment mechanism 27. Both reamer 1 and adjustment mechanism 27 are in a non-deformed state. In this state the adjustment mechanism 27 and control element 30 together provide sufficient structural support for the flap 26 being enclosed by the recess 22 being in a desired position and leading to a desired cutting depth of the reamer 1. In a second state the control element 30 is pushed by the drilling machine 700 further along the rotation axis 6 by a distance d (see Fig. 21) and hence pressing against the V shaped support. The applied force deforms the V shaped support leading to a V with a larger angle and thereby forcing the flap 26 to bend outwards by a distance r. This limits the cutting depth of the reamer 1. Cutting might be even prevented completely if the flap 26 is bend out far enough. Hence control of the pin position along the rotation axis 6 can be used to control the cutting power of the reamer 1.

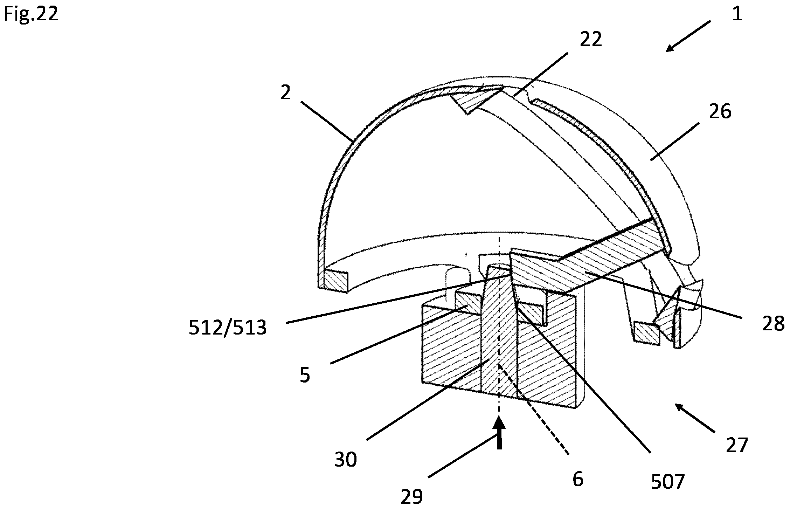

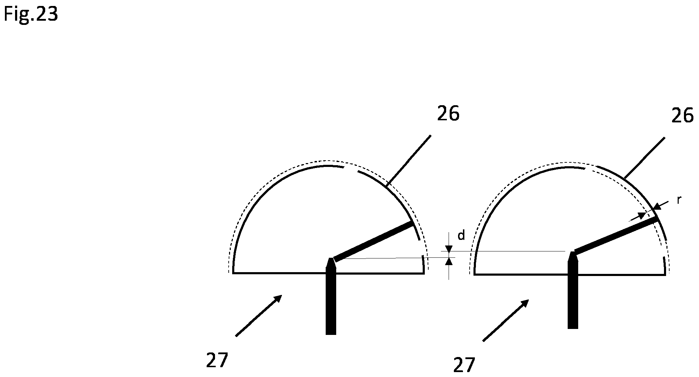

According to Fig. 22 and 23, the adjustment mechanism 27 might connect to the inner surface of the flap 26 and reaches to the reamer centre. The adjustment mechanism 27 has a first contact surface 512 which e.g. might be conically shaped which is designed to be in contact with a second contact surface 513 of the control element 30 which also might be conically shaped. The two contact surfaces 512, 513 are designed as such that when the control element 30 is pushed along the rotation axis 6 by a defined distance d (see Fig. 23) a pressure force oriented in approximately radial direction is generated which pushes the adjustment mechanism 27 by a defined distance r in approximately radial direction and hence reduces the cutting depth of the reamer 1 by the distance r. Hence control of the control element 30 position along the rotation axis 6 can be used to control the cutting power of the reamer 1. An advantage of this design is that the adjustment mechanism 27 only spans half the reamer 1 and hence more room for storage of cut bone material is present.

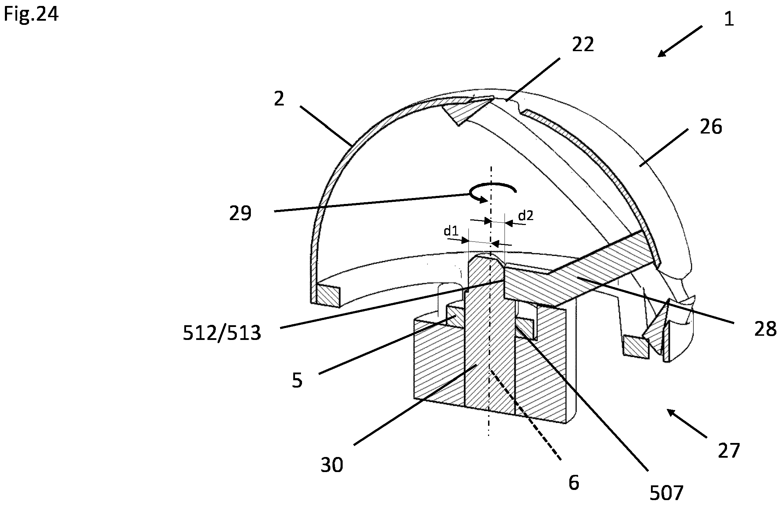

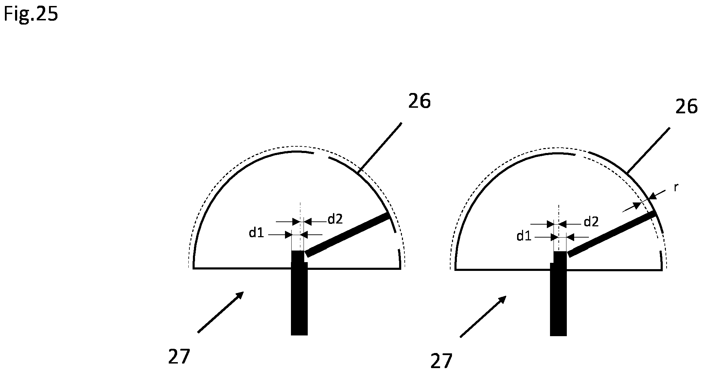

According to Fig. 24 and 25, the first contact surface 512 of the adjustment mechanism 27 is of a non-concentric shape. The control element 30 does not move along the rotation axis 6, but can freely rotate around this rotation axis 6. Due to the non-concentric shape of the first contact surface 512 the adjustment mechanism 27 is pushed by a distance d1 minus d2 (see Fig. 25) which results in a defined lift r of the flap 26.

According to Fig. 26, the reamer 1 might be equipped with the adjustment mechanism 27, but the reamer 1 is designed to have no flap, but a rigid portion enclosed by the recess 22 (which does not allow for significant deformation). The reamer 1 further has a cut preventing component 28 being of cylindrical general shape and comprising a circular shaped edge 603 which is positioned beneath (in direction to the reamer centre) the cutting edge 9 of the cutting section 8. The cut preventing component 28 might be rigidly fixated to the blade element 7 at a connection area 606 away from the circular shaped edge 603 and being separated by a deformable section 607. When pushed out, the control element 30 presses against a lever component 609 which elevates the cut preventing component 28 in such a way that its circular shaped edge 603 reaches and/or overtops the cutting edge 9 of the cutting section 8 in radial direction. In this state the cut preventing component 28 blocks the cutting section 8 to effectively penetrate the bone material and hence prevents the reamer 1 from cutting bone.

The cut preventing component 28 might also be moved through a separate opening in the hemispherical portion 3 in close approximation of the ring blade (not shown). It might be of any shape suitable to overtop the cutting edge 9 and thereby lift of the cutting edge 9 from the bone surface and prevent it from cutting bone. When formed as shown in Fig. 26 the cut preventing component 28 contacts the leading edge of the cutting section 8. In this design it is sufficient to lift the cut preventing component 28 so that it is in close approximation to the cutting edge 9 (no overtopping required) so that it blocks/hinders bone chips from entering into the recess 22 and hence temporarily glut the cutting section hindering the blade element 7 from cutting as long as the cut prevention component 28 is in the lift up state.

In another option (not shown) the adjustment mechanism alters the height of the ring blade so that the cutting edge is or is not protruding the hemispherical portion of the reamer.

Drilling machine and arrangement

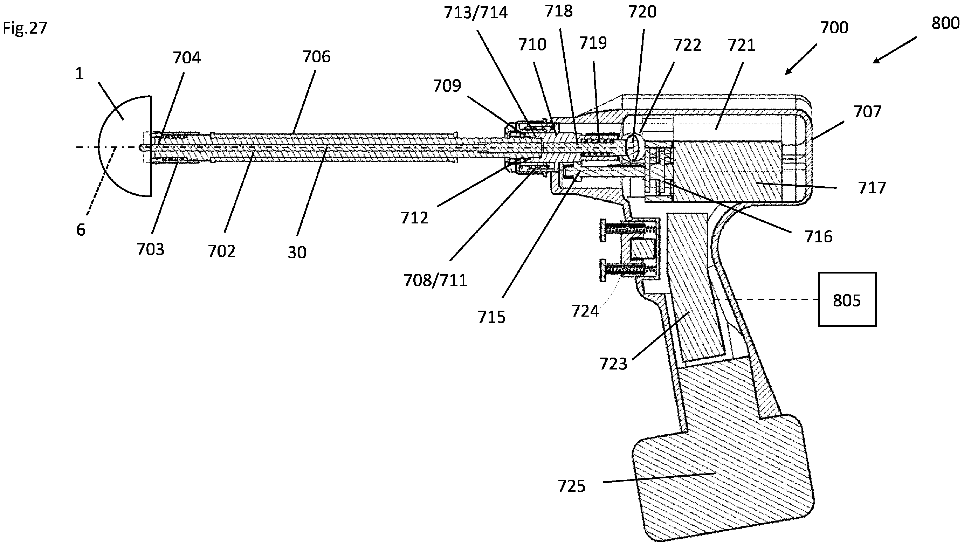

Fig. 27 shows the orthopeadic drilling machine 700 comprising: A shaft 702 connectable to the connector portion 5 of the reamer 1. A main drive 717 for rotating the shaft 702. The control element 30, preferably as a pin in the shaft 702 or a sleeve around the shaft, wherein the control element 30 is adapted to transmit the control movement 29 to the reamer 1 of any embodiment by linear movement and/or rotational movement of the control element 30 relative to the shaft 702. And an auxiliary drive 721 for linear moving and/or rotating the control element 30.

Fig. 27 shows the reamer 1 connected to a shaft 702 (reamer adaptor) of the drilling machine 700 by a first fast connector mechanism 703. The shaft 702 comprises a centre hole 704 along the reamer rotation axis 6 housing the control element 30 in the assembled state. The control element 30 can move in axial direction within the shaft 702 when actuated. The shaft 702 further comprises a handle portion 706, which can rotate freely around the rotation axis and an adapter portion which is connected to the rest of the drilling machine 700 by a second fast connector mechanism 708. The second fast connector mechanism 708 allows for transmission of both the rotation momentum as well as an axial directed force actuating the control element 30. A set of metal spheres 709 are placed in holes of a rotating element 710 which are symmetrically arranged around the rotation axis. In a closed state of the second fast connector mechanism 708 these metal spheres 709 are pressed by a coil mechanism 711 into the holes and into a groove 712 of the shaft 702, locking the axial position of the shaft 702 relative to the drilling machine 700.

The rotation momentum is transmitted by a form fit. The end part of the shaft 702 is therefore designed as a hexagonal 713 which is inserted into a hexagonal hollow 714 of the rotating element 710.

The reamer drill shaft is actuated by a spur gear 715 which is connected to a planetary drive box 716 being actuated by a first electric rotary motor, referred to as main drive 717. The gear system converts the motor torque and spin frequency into a desired torque and spin frequency desired for reaming.

Axial forces are transmitted to the control element 30 by a piston 718. The piston 718 is free to slide within a centre hole of the rotating element 710 and connects to a coil spring 719 which pushes the piston against a camshaft 720. In a first position of the camshaft 720 the piston 718 is in a non-pushed position where no force is transmitted to the control element 30. In a second position the camshaft 720 pushes the piston 718 in axial direction against the control element 30 and translates both components by a defined distance d in axial direction.

The actuation force is provided by a second electric rotary motor, referred to as auxiliary drive 721. A worm gear 722 converts the motor torque and spin frequency into a desired torque and spin frequency to actuate the camshaft 720. Further the drilling machine 700 is equipped with at least two rotation position sensors which track the orientation of the rotating element 710 (same rotation as shaft 702) and the orientation of the camshaft 720.

The electric motors and position sensors are connected to a control unit 723. Further the drilling machine 700 comprises finger triggers 724 whose position is tracked by a sensor which is connected to the control unit 723. The control unit 723 further allows communication with external components such as a surgical navigation system by wire or wireless. Energy to the drilling machine 700 is provided either by a battery pack 725 or a power cable.

Fig. 27 shows an arrangement 800 for milling the hemispherical cavity 901 in the bone 900, comprising: The orthopeadic drilling machine 700 with housing 707, the orthopeadic reamer 1 , adapted to adjust the cutting depth by the control movement of the control element driven by the auxiliary drive 721 of the drilling machine 700, and a controller arrangement 805 adapted: (A) to determine or receive at least one angle range 806, which is less than 360° and higher than 0°, (B) to determine or receive the actual rotational angle of the reamer 1 during milling, (C) and to control the auxiliary drive 721 to reduce the cutting depth during the reamer 1 rotates through (within) the angle range 806, wherein the cutting depth in the angel range 806 is reduced compared to a cutting depth during rotation outside the angle range 806.

The controller arrangement 805 can comprise one computational unit or several connected computational units. Control unit 723 can be part of the controller arrangement 805. The controller 805 can be outside or inside the housing 707.

The control arrangement 805 is adapted to monitor the orientation of the bone 900 and to control the auxiliary drive 721 in dependency of the angle range and the orientation of the bone 900. The control arrangement 805 is adapted to monitor the orientation of the drilling machine 700 and to control the auxiliary drive 721 in dependency of the angle range and the orientation of the drilling machine 700. The control arrangement 805 is adapted to determine or receive the initial rotational angle of the reamer 1 before the main drive 717 rotates the reamer 1 and to keep track of the actual rotational angle of the reamer 1 by receiving a signal from a rotation position sensor within the drilling machine 700.

The inventive arrangement 800 allows selective reaming by e.g. the following use of the reamer 1: Pose of drilling machine 700 and hip joint is tracked by surgical navigation system or the like. Rotation orientation of the reamer 1 is tracked by rotation sensor of drilling machine 700. So the reamer pose in 3D space and the relative position to the bone is known. A user inputs the angel range of critical bone areas. The controller arrangement 805 calculates the target position for the control element 30 for each rotation orientation of the cutting section 8 based on the angle range/critical bone area. During reaming, the auxiliary drive 721 actuates the control element 30 based on the angle range and depending on the current position of the cutting section 8. So, for each reamer revolution, no cutting or reduced cutting depth is performed on the critical bone area.

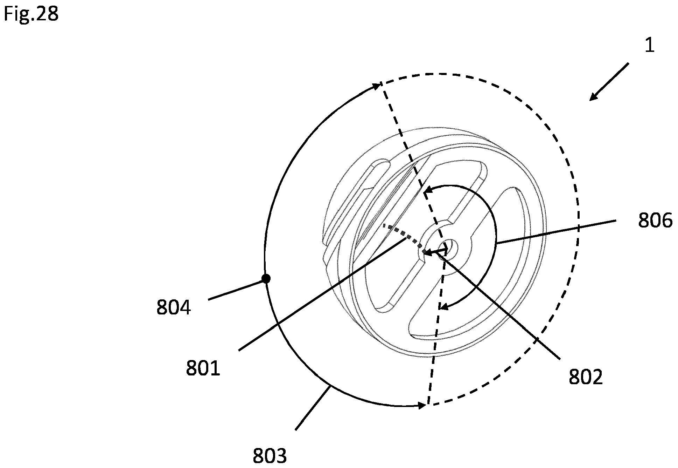

With reference to Fig. 28: Provided that a desired end position of the reamer 1 in respect to the patient's bone 900 is given as input to the controller arrangement 805 a desired reamer path 801 can be computed. During the reaming process the current position of the reamer 1 is compared to the desired position on the calculated target reamer path resulting in a displacement vector 802.

The control element position is then controlled in such a way that cutting is only performed in a defined rotation section 803 but not in the angle range 806. The centre 804 of the rotation section 803 is roughly located in direction of the displacement vector 802. Repeating this operation for each revolution of the reamer 1 allows for directional reaming.

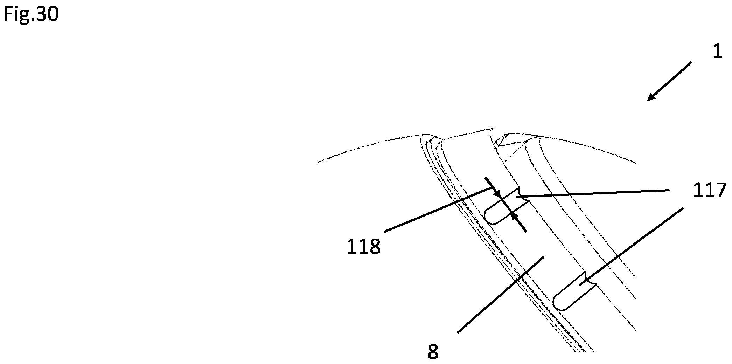

Fig. 29 and 30 disclose features for all above mentioned reamers 1: The sharp cutting edge 9 having following angles, wherein the spherical surface on which the tip of the cutting edge 9 moves when the reamer 1 is used is referred to as sliding plane 115, and the cutting edge 9 is positioned between the sliding plane 115 and the normal vector of the sliding plane 115: the clearance angle 113 is between the sliding plane 115 and the outward facing side of the cutting edge 9; the rake angle 111 is between the normal vector of the sliding plane 115 and the inward facing side of the cutting edge 9; the rake angel; the blade angle 112 is between the inward facing side and the outward facing side of the cutting edge 9. Fig. 29 shows the cutting depth of the reamer 1 as d_{cut}.

According to Fig. 30, the cutting edge 9 of all embodiments can have one or more chip breakers 117 having a width 118 locally protruding from the cutting section 8 to form a groove in the bone 900.

### List of reference signs

1 reamer
2 body
3 hemispherical portion
4 cavity
5 connector portion
6 rotation axis
7 blade element
8 cutting section
9 cutting edge
10 blade axis
11 apex
12 equator
13 virtual cutting sphere
14 midpoint of the hemispherical portion
15 support section
16 area of cross section
17 section angle
18 direction of rotation
19 single cutting area
20 height
21 X-ray absorber
22 curved recess
23 bridge
24 bridge-blade portion
25 reinforcement structure
26 flap
27 adjustment mechanism
28 cut preventing component
28 control arrangement
29 control movement
30 control element
31 intersection portion
32 top blade's bottom point
33 bottom blade's top point
34 apex-blade portion
35 radius
111 rake angle
112 blade angle
113 clearance angle
115 sliding plane
117 chip breaker
118 width
204 connection portion
205 inner surface
206 connecting surface
207 circular shaped edge
212 additional opening
213 element of the connecting portion
214 hole
215 clamping system
216 centring rod
217 groove
218 screw
219 pressure plate
220 crossbar design
221 flat sheet metal
222 plastic parts
223 bridgeback design
226 clip
229 portion enclosed by recess
303 cutting/stamping tool
304 cutting element
305 matrix component
306 matrix component recess
309 second opening
310 second cutting element
311 second matrix component recess
312 conical surface
405 first surface of cylindrical shape
406 the outside surface of the blade element
407 flat surface
408 bottom surface of the blade element
409 circulating overhang
411 alignment features
507 hole
512 first contact surface
513 second contact surface
603 circular shaped edge
606 connection area
607 deformable section
609 lever component
700 drilling machine
702 shaft
703 first fast connector mechanism
704 centre hole
706 handle portion
707 housing
708 second fast connector mechanism
709 metal spheres
710 rotating element
711 coil mechanism
712 groove
713 hexagonal
714 hexagonal hollow
715 spur gear
716 planetary drive box
717 main drive
718 piston
719 coil spring
720 camshaft
721 auxiliary drive
722 worm gear
723 control unit
724 finger triggers
725 battery pack
800 arrangement
801 reamer path
802 displacement vector
803 rotation section
804 centre
805 controller arrangement
806 angle range
900 bone
901 hemispherical cavity
902 uncut portion

## Claims

1. Orthopeadic reamer (1), preferably acetabular reamer, for milling a hemispherical cavity (901) in a bone (900), preferably adapted for single use, comprising
• a body (2),
∘ having a hemispherical portion (3) spanning a cavity (4), preferably to store cut bone debris, adapted for attachment of a drilling machine (700), preferably via a connector portion (5) of the body, to rotate the reamer (1) around a rotation axis (6),
• and at least one ring-shaped blade element (7)
∘ having a curved cutting section (8) with a sharp cutting edge (9),
∘ wherein the ring-shaped blade element (7), preferably each ring-shaped blade element, is embedded in the hemispherical portion (3),
∘ and wherein a blade axis (10) of the ring-shaped blade element is defined perpendicular to a radius (35) of the ring-shaped blade element (7), and the blade axis (10), preferably of each ring-shaped blade element, is inclined to the rotation axis (6).

2. Orthopeadic reamer according to claim 1, wherein the blade axis (10) intersects the midpoint (14) of the hemispherical portion (3).

3. Orthopeadic reamer according to claim 1 or 2, wherein the ring-shaped blade element (7) is a closed ring extending over a ring angle of 360° around the blade axis (10), or is an open ring extending over a ring angle of at least 200°, preferably at least 250°, around the blade axis (10).

4. Orthopeadic reamer according to any preceding claim, wherein the ring-shaped blade element (7) is circular or elliptical.

5. Orthopeadic reamer according to any preceding claim, wherein the cutting section (8) of the ring-shaped blade element (7) extends over a section angle (17) between 100° and 220°, preferably between 160° and 200°, around the blade axis (10).

6. Orthopeadic reamer according to any preceding claim, wherein the cutting section (8) of the ring-shaped blade element (7) merges into a support section (15) of the ring-shaped blade element (7), so that the circumference of the ring-shaped blade element (7) is formed by the cutting section (8) and by the support section (15).

7. Orthopeadic reamer according to claim 6, wherein the support section (15) has no cutting edge and is preferably positioned as to have no contact to the bone.

8. Orthopeadic reamer according to any preceding claim, wherein the reamer (1) is designed as one blade reamer having only the one ring-shaped blade element (7); preferably, the reamer (1) has no other cutting elements and/or no other grinding elements.

9. Orthopeadic reamer according to any preceding claim, wherein a single cutting area (19) is defined on the hemispherical portion (3) of the body (2), wherein only one blade element (7) is disposed in the single cutting area (19), wherein the single cutting area (19) is a full circumference ring area that extends from the equator (12) of the hemispherical portion (3) in direction of the apex (11), wherein a height (20) of the single cutting area (19) is measured from the equator (12) in direction of the apex (11) parallel to the rotation axis (6) of the reamer (1) and the height (20) is at least 0,2*R, preferably at least 0,25*R, with R being the radius of the hemispherical portion (3); preferably wherein the single cutting area (19) is designed as a smooth, spherical surface to support and guide the reamer (1) gliding on the bone.

10. Orthopeadic reamer according to anyone of claims 1 to 8, wherein the reamer (1) is designed as multi blade reamer having two, three, or four of the one ring-shaped blade elements (7), wherein the cutting sections (8) of all ring-shaped blade elements (7) are arranged on one half of the hemispherical portion (3) so that the opposing area of the hemispherical portion (3) can contact the bone; preferably wherein the opposing area is designed as a smooth, spherical surface to support and guide the reamer (1) gliding on the bone.

11. Orthopeadic reamer according to any preceding claim, wherein the hemispherical portion (3) having at least one curved recess (22), preferably slit formed.

12. Orthopeadic reamer according to claim 11, wherein a section, referred to as flap (26), of the hemispherical portion (3) on the concave side of the curved recess (22) is adjustable in order to adjust the cutting depth of the blade element (7).

13. Orthopeadic reamer according to claim 12, comprising an adjustment mechanism (27), preferably positioned in the cavity (4) of the hemispherical portion (3), wherein the adjustment mechanism (27) is adapted to lift and/or lower the flap (26) relative to the blade element (7) in order to adjust the cutting depth of the blade element (7).

14. Orthopeadic reamer according to claim 11, comprising a cut preventing component (28), and an adjustment mechanism (27), wherein the cut preventing component (28) is movable through the recess (22) or a separate opening in the hemispherical portion (3) by the adjustment mechanism (27), so that the cut preventing component (28) can overtop the cutting edge (9) to prevent the reamer (1) from cutting.

15. Orthopeadic reamer according to claim 13 or 14, wherein the adjustment mechanism (27) has a control arrangement (28), which is adapted to be operated by a control movement (29) of a control element (30) of the drilling machine (700).
